# EUROPEAN PATENT APPLICATION

(11) **EP 1 477 483 A1**
(43) Date of publication of application: **17.11.2004**
(21) Application number: 03705314.7
(22) Date of filing: 19.02.2003
(51) Int. Cl.: C07D 307/58, A61K 31/593, A61P 3/02, A61P 3/10, A61P 3/14, A61P 5/18, A61P 9/12, A61P 11/00, A61P 17/02, A61P 17/06, A61P 17/10, A61P 17/14, A61P 19/08, A61P 19/10, A61P 29/00, A61P 35/00, A61P 43/00

(54) **VITAMIN D3 DERIVATIVES AND REMEDIES USING THE SAME**

(30) Priority: 20.02.2002 JP 2002043042
(71) Applicant: TEIJIN LIMITED, Osaka-shi Osaka 541-0054 (JP)
(72) Inventor: Takenouchi, Kazuya, c/o Teijin Ltd, Tokyo Res. Cte, Hino-shi, Tokyo 191-0065 (JP); Anzai, Miyuki, c/o Teijin Ltd, Tokyo Res. Center, Hino-shi, Tokyo 191-0065 (JP); Manabe, Kenji, c/o Teijin Ltd, Tokyo Res. Center, Hino-shi, Tokyo 191-0065 (JP); Ishizuka, Seiichi, c/o Teijin Ltd, Tokyo Res. Cter, Hino-shi, Tokyo 191-0065 (JP); Miura, Daishiro, c/o Teijin Ltd, Tokyo Res. Center, Hino-shi, Tokyo 191-0065 (JP)
(74) Representative: Hallybone, Huw George
(86) International application number: PCT/JP2003/001775
(87) International publication number: WO 2003/070716

(57) **Abstract**

A vitamin D₃ derivative expressed by the following general formula (1) 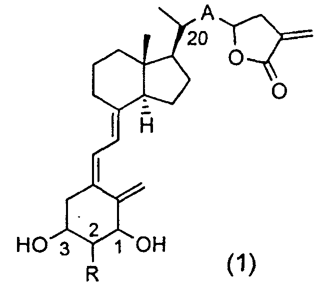 [wherein, R is a hydrogen atom or a methyl group, and A is a single bond, -CH₂-, -CH=CH-, -CH₂-CH=CH-, -CH=CH-CH=CH-, -C=C- or -CH₂-C≡C-; and a compound in which R is an hydrogen atom, A is -CH₂-, the steric configuration at the 1-position is (S)-configuration, and the steric configuration at the 3-position is (R)-configuration is excluded] or a pharmaceutically permissible solvate thereof.

The compound can be an effective ingredient of a treating agent for osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia or Paget's disease of bone.

## Description

### Technical Field

The present invention relates to vitamin D₃ derivatives useful as pharmaceutical products or pharmaceutically permissible solvates thereof, treating agents using same and pharmaceutical compositions containing same. More particularly, the invention relates to 1α-hydroxyvitamin D₃ derivatives or pharmaceutically permissible solvates thereof, treating agents containing same as an active ingredient and effective for osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia or Paget's disease of bone, and pharmaceutical compositions containing same.

### Background Art

Active vitamin D₃ derivatives have variegated actions such as calcium absorption-stimulating activity in the small intestine, bone turnover-controlling activity, immunoregulatory activity, cytostatic activity and cell differentiation inducting activity. Applications of these derivatives to treating agents using these actions for various diseases, for example, osteoporosis, malignant tumor, psoriasis, hyperparathyroidism, an inflammatory respiratory disease, rheumatoid arthritis, growth onset type diabetes mellitus, hypertension, alopecia, acne, dermatitis, etc. are being studied, and they are actually applied to such treating agents.

Out of these treating agents, the present conditions of the study of a treating agent for hyperparathyroidism are as follows. It is known that various diseases are caused by the disorders in the abnormal production quantity of parathyroid hormone (hereafter, PTH) in vivo. Examples of the diseases include primary and secondary hyperparathyroidism caused by abnormal hyper-production of PTH. Primary hyperparathyroidism is a systemic disease caused by PTH hypersecretion from one or more parathyroids, and it is known that about 90 % of the patients suffers from parathyroid adenoma. Secondary hyperparathyroidism is a disease which develops in a patient with chronic renal failure owing to conditions in which parathyroids grown as a result of metabolic disturbance of active vitamin D, calcium and phosphorus exhibit resistance against 1α, 25-dihydroxyvitamin D₃ of physiological concentration, and the hyperplasia of parathyroids further progresses to secrete PTH excessively. Bone resorption is accentuated by excess PTH, and resultingly, ostealgia and arthralgia are observed in a number of cases. Further, symptoms at other sites than bone such as ectopic calcification of the soft tissue and the arterial wall caused by hypercalcemia and hyperphosphatemia are observed. Various kinds of mechanisms of occurrence are proposed for secondary hyperparathyroidism, and especially important one is inhibition of 1α-hydroxylase activity on vitamin D due to renal function degeneracy. When the activity of the enzyme is inhibited, the serum concentration of 1α, 25-dihydroxyvitamin D₃ is lowered, failure of calcium absorption from the small intestine and hyperphosphatemia due to the lowering of phosphorus excretion from the kidneys occur, and as the result, the state of hypocalcemia develops. It is considered that when this state continues, PTH secretion is actuated, and secondary hyperparathyroidism progresses. Accordingly, it is desirable to administer a compound having pharmacological activity like 1α, 25-dihydroxyvitamin D₃ as a treating agent for secondary hyperparathyroidism. As such compounds, 1α, 25-dihydroxyvitamin D₃ itself and 1α-dihydroxyvitamin D₃ are known, and they exhibit high efficacy. However, in a patient to which such a drug has been administered for a long period, the sensitivity to 1α, 25-dihydroxyvitamin D₃ is lowered to make it difficult to suppress hypersecretion of PTH by the administration of normal amount of the drug (vitamin D resistance). Against this problem, Slatopolsky et al. succeeded in suppressing PTH excretion from parathyroids by intermittent intravenous administration of large amount of 1α, 25-dihydroxyvitamin D₃, so called a pulse therapy (J. Clin. Invest., 74, 2136-2143 (1984)). However, this pulse therapy of active vitamin D is apt to cause hypercalcemia due to the administration of a large quantity of a 1α, 25-dihydroxyvitamin D₃ drug, and hence a 1α-hydroxyvitamin D₂ drug (WO96/31215 specification), a 19-nor-1α, 25-dihydroxyvitamin D₂ drug (WO97/02826 specification), or a 22-oxa-1α, 25-dihydroxyvitamin D₃ drug (JP-A 3-7231 (JP-A means Japanese unexamined patent publication)), which has weaker calcium metabolic activity than the 1α, 25-dihydroxyvitamin D₃ drug, is presently administered as a treating agent for treating vitamin D-resistant secondary hyperparathyroidism. However, although the calcium metabolic activities of these vitamin D₃ derivatives are weaker than 1α, 25-dihydroxyvitamin D₃, they still have calcium metabolic activity. As a result, the suppressive activity of PTH production is not sufficiently separated from calcium metabolic activity, and hypercalcemia develops often as adverse effect (Nephrol. Dial. Transport, 11, 121-129 (1996)). Therefore, the therapies using these drugs are not satisfactory from view point of the occurrence of adverse effect. Consequently, a drug which strongly suppresses PTH excretion without causing hypercalcemia, if any, has expectation of more effective therapeutic effect.

On the other hand, hypercalcemia develops by the sthenia of vitamin D production due to diseases such as tumors or hyperparathyroidism. Since it is known that the blood calcium concentration is increased by the action of active vitamin D₃, a compound which is antagonistic against the action of active vitamin D₃, that is, a vitamin D₃ antagonist, is considered to be effective for treating hypercalcemia.

Further, it is thought that the vitamin D₃ antagonist is effective also as a treating agent for Paget's disease of bone. Paget's disease of bone is a disease of unknown etiology in which bone resorption is abnormally accelerated in the pelvis, the thighbone, the skull, etc., and consequently, symptoms such as bone deformation and ostealgia develop. Treating agents presently used for Paget's disease of bone are bisphosphonate drugs and calcitonin drugs, which are also used as osteoporosis treating agents. But, the former has poor compliance since the necessary administration dose is 4 to 5 times the administration dose for an osteoporosis patient, and the later has a disadvantageous point that sufficient suppression of bone resorption is not exhibited. Further, these drugs are symptomatolytic agents resting on the basis of the bone resorption suppressing activity of the agents and can not completely cure the disease. It has recently been made clear that precursor cells of osteoclast collected from a patient of Paget's disease of bone have 1α, 25-dihydroxyvitamin D₃ receptors, and the sensitivity of the precursor cells to 1α, 25-dihydroxyvitamin D₃ is stronger by about 10 to 100 times that of the precursor cells of osteoclast in a normal person (J. Bone Miner. Res., 15, 228-236 (2000)). Further, the blood concentration of 1α, 25-dihydroxyvitamin D₃ in a patient of Paget's disease of bone being same as in a normal person, the sthenia of bone resorption by endogenous 1α, 25-dihydroxyvitamin D₃ is supposed to play an important role to the onset of Paget's disease of bone. Consequently, a compound which suppresses the action of 1α, 25-dihydroxyvitamin D₃ on the precursor cells of osteoclast, that is, a compound like a vitamin D antagonist, can suppress the bone resorption activity accelerated in a patient of Paget's disease of bone more completely, and it can be expected that the compound has therapeutic effect superior to a presently used bone resorption-suppressing agent.

Further, although the specification of WO95/33716 and the specification of WO00/24712 have disclosed compounds having an α-methylenelactone structure as a D-ring side chain of vitamin D₃, these compounds are excluded from the compounds of the present invention, and as for the compounds described in these specifications, there is no description nor suggestion regarding whether they have PTH production-suppressing activity and vitamin D₃ antagonist activity, or not.

Furthermore, JP-A 11-116551 and the specification of WO98/50353 have disclosed compounds having a methyl group as a 2-position substituent of vitamin D₃. However, in these compounds, the D-ring side chain of vitamin D₃ is a 1α, 25-dihydroxyvitamin D₃-type (6-hydroxy-6-methylheptan-2-yl), and they are different from the compounds of the present invention which have an α-methylenelactone structure. There are no description nor suggestion whether the compounds described in the above-mentioned JP-A 11-116551 and specification of WO98/50353 have PTH production-suppressing activity and vitamin D₃ antagonist activity, or not.

### Disclosure of the invention

It is an object of the present invention to provide new vitamin D₃ derivatives effective as a treating agent for osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia, Paget's disease of bone or the like, or pharmaceutically permissible hydrates thereof.

Another object of the present invention is to provide methods for treating osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia, Paget's disease of bone or the like by administering these vitamin D₃ derivatives or pharmaceutically permissively solvates thereof.

Still another object of the present invention is to provide pharmaceutical compositions containing these vitamin D₃ derivatives or pharmaceutically permissible solvates thereof as active ingredients.

These objects of the present invention can be achieved by vitamin D₃ derivatives expressed by the following general formula (1), 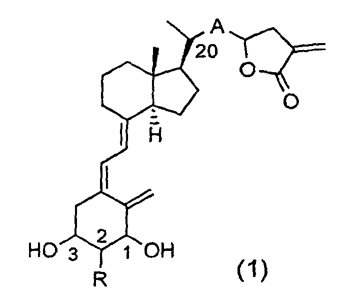 [wherein, R is a hydrogen atom or a methyl group, and A is a single bond, -CH₂-, -CH=CH-, -CH₂-CH=CH-, -CH=CH-CH=CH-, -C=C- or -CH₂-C≡C-; and a compound in which R is a hydrogen atom, A is -CH₂-, the steric configuration at the 1-position is (S)-configuration, and the steric configuration at the 3-position is (R)-configuration is excluded] or pharmaceutically permissible solvates thereof.

When an asymmetric carbon is contained in a structure of a compound in the above formula (1), the steric configuration may be either (S)-configuration or (R)-configuration as far as it is not particularly specified. When the above formula (1) has a double bond, the geometrical configuration may be either (E)-configuration or (Z)-configuration as far as it is not particularly specified. Further, mixtures of an arbitrary ratio of these isomers are also included in the present invention.

Further, objects of the present invention can be achieved by administering vitamin D₃ derivatives expressed by the above formula (1) or pharmaceutically permissible solvates thereof in therapeutically effective amounts as active ingredients to patients of osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia or Paget's disease of bone.

Furthermore, objects of the present invention are achieved by pharmaceutical compositions comprising a vitamin D₃ derivative expressed by the above formula (1) or a pharmaceutically permissible solvate thereof, and a pharmaceutically permissible support.

### Best Mode for Carrying Out the Invention

In the above formula (1), R is a hydrogen atom or a methyl group.

In the above formula (1), A is a single bond, -CH₂-, -CH=CH-, -CH₂-CH=CH-, -CH=CH-CH=CH-, -C=C- or -CH₂-C≡C-; and -CH₂-, - CH=CH-, -CH=CH-CH=CH-, -C≡C- or -CH₂-C≡C- is preferable; and -CH₂- or -CH=CH- is particularly preferable.

In the above formula (1), when an asymmetric carbon is contained in the structure of a compound, the steric configuration may be either (S)-configuration or (R)-configuration as far as it is not particularly specified. Especially, derivatives having (S)-configuration at the 1-position and (R)-configuration at the 3-position, and derivatives having (S)-configuration at the 1-position and (S)-configuration at the 3-position are preferable, and derivatives having (S)-configuration at the 1-position and (R)-configuration at the 3-position are most preferable. Further, when a methyl group exists at the 2-position, the steric configuration of the 2-position is preferably (S)-configuration.

In the above formula (1), when a double bond is contained, the geometric configuration may be either (E)-configuration or (Z)-configuration.

Further, mixtures of an arbitrary ratio of these isomers are also included in the present invention.

A vitamin D₃ derivative of the present invention may be converted into a pharmaceutically permissible solvate at need. Examples of the solvent include water, methanol, ethanol, propyl alcohol, isopropyl alcohol, butanol, t-butanol, acetonitrile, acetone, methyl ethyl ketone, chloroform, ethyl acetate, diethyl ether, t-butyl methyl ether, benzene, toluene, DMF, DMSO, etc. Especially, water, methanol, ethanol, propyl alcohol, isopropyl alcohol, acetonitrile, acetone, methyl ethyl ketone and ethyl acetate may be cited as preferable examples.

The compounds shown in Table 1 may be cited as preferable concrete examples of a vitamin D₃ derivative expressed by the formula (1) of the present invention. When an asymmetric carbon is contained in the structure of a compound in the table, the steric configuration may be either (S)-configuration or (R)-configuration as far as it is not particularly specified. When a double bond is contained in a compound of the above formula (1), the geometrical configuration may be either (E)-configuration or (Z)-configuration as far as it is not particularly specified. Further, mixtures of an arbitrary ratio of these isomers are also included in the present invention.

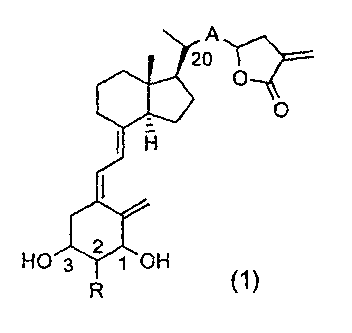

**Table 1**

| Compound No. | A | R |
|---|---|---|
| 11 | Single bond | hydrogen atom |
| 21 (note) | -CH₂- | hydrogen atom |
| 22 | -CH₂- | methyl group |
| 31 | -CH=CH- | hydrogen atom |
| 32 | -CH=CH- | methyl group |
| 41 | -CH₂-CH=CH- | hydrogen atom |
| 51 | -CH=CH-CH=CH- | hydrogen atom |
| 61 | -C≡C- | hydrogen atom |
| 71 | -CH₂-C≡C- | hydrogen atom |
| (note) The derivatives having (S)-configuration in the steric configuration at the 1-position and (R)-configuration at the 3-position are excluded. | | |

A vitamin D₃ derivative expressed by the above formula (1) can be manufactured, for example, as shown below. That is, an aldehyde compound expressed by the following formula (2) is converted into a lactone compound expressed by the following formula (3), and this compound is subjected to a coupling reaction with an ene-yne compound expressed by the following formula (4) in the presence of a palladium catalyst and successively to the deprotection of the protective group of the hydroxyl group (Scheme 1). 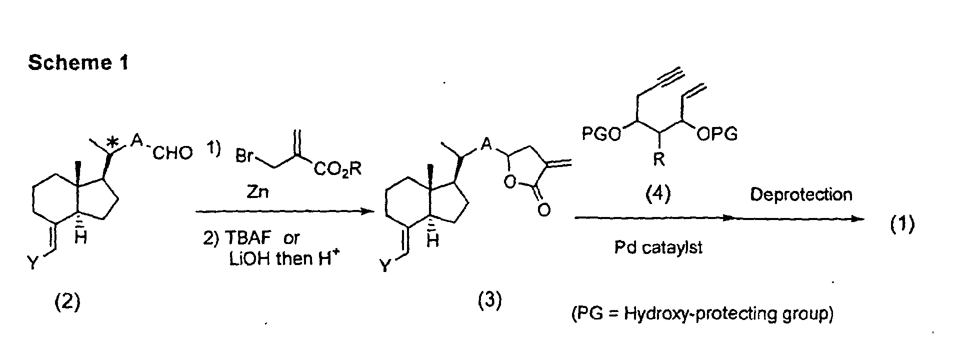 [In the formulae (2) and (3), A is defied same as in the above formula (1) and Y is a bromine atom or an iodine atom. In the above formula (4), R is defined same as in the above formula (1); and PG expresses a protective group of the hydroxyl group, which includes acetyl group, a tetrahydro-4H-pyran-2-yl group, a tri(alkyl/aryl)silyl group such as a trimethylsilyl group, a triethylsilyl group, a t-butyldimethylsilyl group (TBS) or a t-butyldiphenylsilyl group, or the like].

An aldehyde compound (2) used here in which the steric configuration of the carbon atom marked with * is (R)-configuration [in the case where A is a single bond, the steric configuration is (S)-configuration] can be obtained as shown below. That is, compounds in which A is a single bond, -CH=CH-, -C≡C- or -CH=CH-CH=CH-, A is -CH₂- or -CH₂-CH=CH-, and A is -CH₂-C≡C- can be obtained by combining known methods as shown in the below-mentioned scheme 2, scheme 3 and scheme 4, respectively. 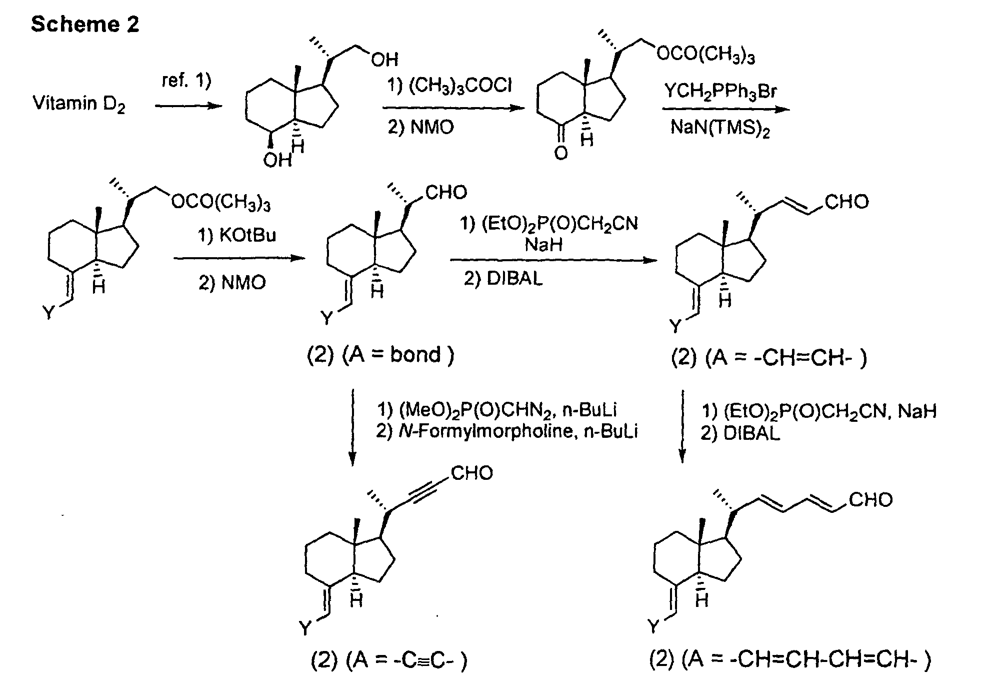 ref. 1) *J. Org. Chem*., **1986**, *51*, 1264.

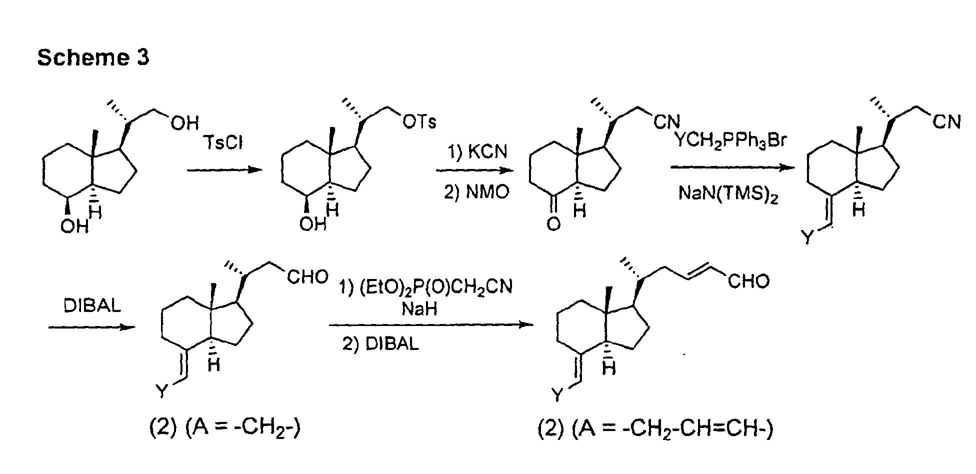

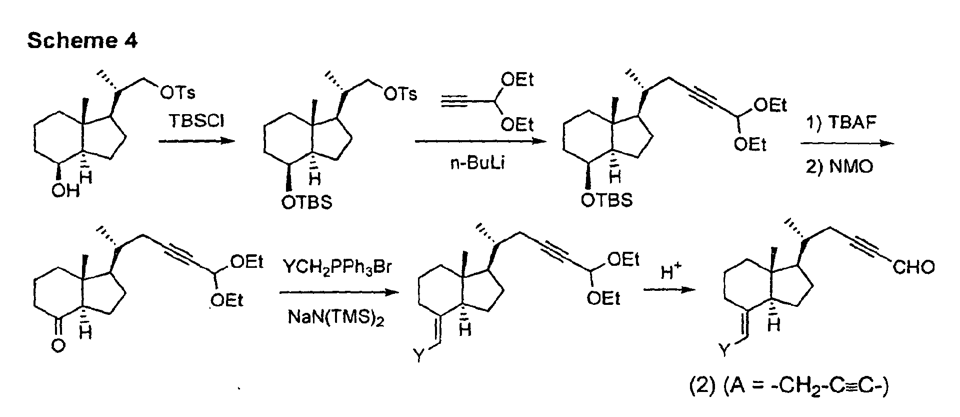

Further, compounds in which the steric configuration of the carbon atom marked with * is (S)-configuration [in the case where A is a single bond, the steric configuration is (R)-configuration] in formula (2), can be obtained, for example, by the method shown in the below-mentioned scheme 5 by using an intermediate aldehyde obtained in scheme 2.

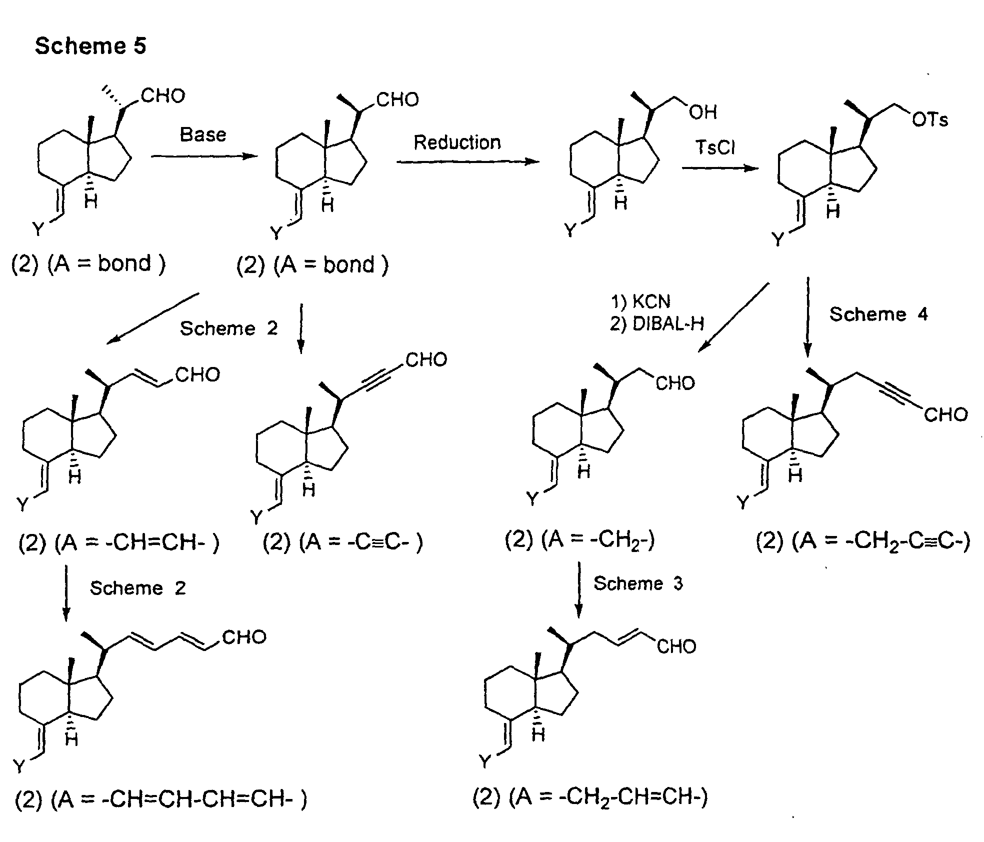

The ene-yne compounds (4) used here can be obtained according to a literature-described method. For example, in the case of R of a hydrogen atom, the method is described in J. Am. Chem. Soc, 114, 9836-9845 (1992) and Tetrahedron Lett., 35, 8119-8122 (1994) by Trost et al.; and in the case of R of a methyl group, in J. Med. Chem., 43, 4247-4265 (2000) by Konno et al.

The conversion of a compound expressed by formula (2) to the compound expressed by formula (3) can be conducted, for example, by allowing the compound expressed by formula (2) to react with a bromomethylacrylate in the presence of zinc and treating the obtained hydroxy ester compound with tetra-n-butylammonium fluoride (TBAF), or by subjecting the ester compound to hydrolysis with a base and successively treating the product with diluted hydrochloric acid.

A coupling reaction of a compound expressed by formula (3) with a compound expressed by formula (4) can be carried out, for example, by a method described in J. Am. Chem. Soc., 114, 9836-9845 (1992) by Trost et al.

The deprotection reaction of the protective group of the hydroxyl group on the obtained coupling product can be carried out according to a known method (for example, see Protective Groups in Organic Synthesis Third Edition, John Willey & Sons, Inc. 1999, Greene et al.). Further specifically, when the protective group is an acetyl group, the reaction can be carried out by a common alkali hydrolysis reaction, or a treatment with potassium cyanide, ammonia-methanol or the like. When the protective group is a methoxymethyl group or a tetrahydro-4H-pyran-2-yl group, the reaction can be carried out under acidic conditions, for example, by using hydrochloric acid, acetic acid, trifluoroacetic acid or the like, or pyridinium p-toluenesulfonate (PPTS) or the like. When the protective group is a tri(alkyl/aryl)silyl group such as a trimethylsilyl group, a triethylsilyl group, a t-butyldimethylsilyl group, a t-butyldiphenylsilyl group or the like, the reaction can be carried out according to a known method (for example, Tetrahedron, 20, 4609-4619 (1987) by Caverley), and as the deprotecting agent, for example, TBAF, PPTS, p-toluenesulfonic acid, hydrogen fluoride, camphorsulfonic acid, hydrochloric acid, sulfuric acid, an agent comprising a combination of a tetrafluoroborate alkali metal salt and sulfuric acid, or the like can be used. Examples of an organic solvent to be used in the reaction include a halogen-containing solvent such as methylene chloride, chloroform or carbon tetrachloride, a hydrocarbon solvent such as hexane or toluene, an ether solvent such as tetrahydrofuran or dioxane, a water-soluble solvent such as N,N-dimethylformamide or acetonitrile, a mixed solvent of them, etc. The solvent may be selected in consideration of the solubility and the reactivity of the compound. The reaction temperature generally ranges from -20°C to the boiling point of the solvent. The reaction time depends on the dehydrating agent, deprotecting agent, reaction solvent and reaction temperature used, and it is commonly preferable that the reaction is continued until the starting material disappears when determined by using an analytical means such as thin layer chromatography.

The manufacturing of a vitamin D₃ derivative expressed by the above formula (1) in which R is a hydrogen atom can be conducted, for example, as shown in the below-mentioned scheme 6: Compound (10) is produced from Compound (5) obtained from vitamin D₂ by combining a photoisomerization reaction and a conversion reaction of the 20-position aldehyde; and the protective groups of the hydroxyl group of Compound (10) are deprotected. 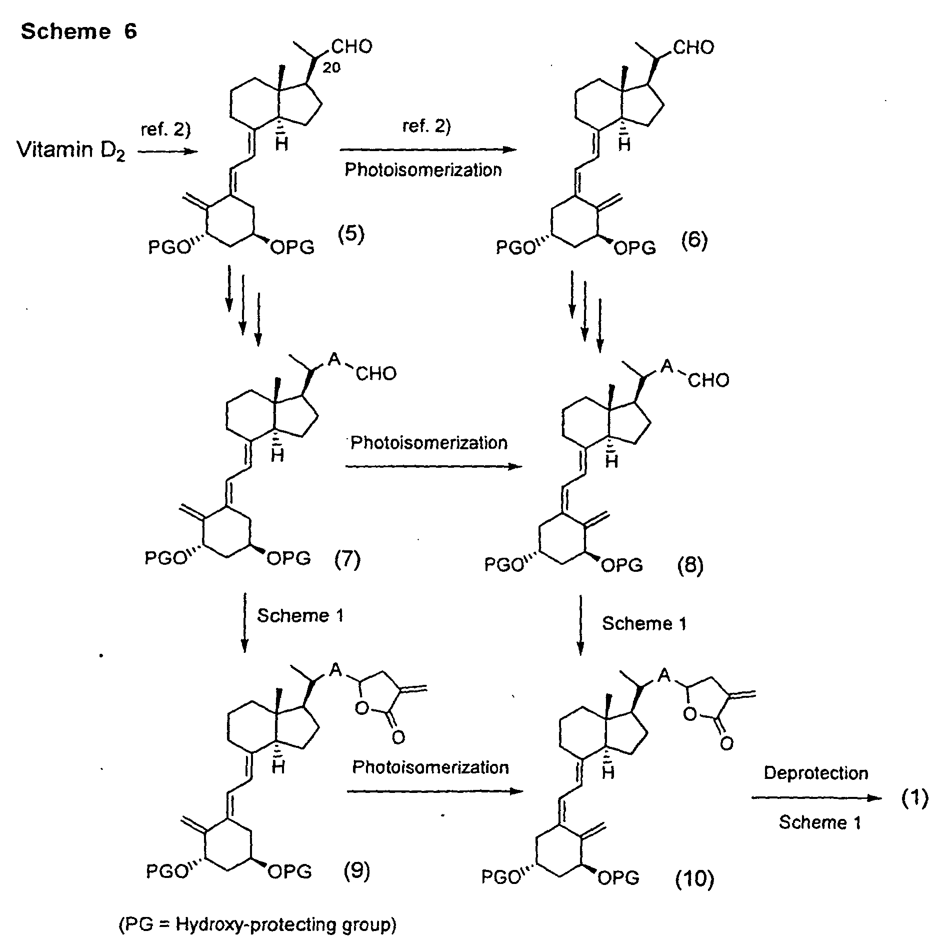 ref. 2) *Tetrahedron,* **1987**, *20*, 4609.
[In the formulae (5) to (10), A is defied same as in the above formula (1); and PG expresses a protective group of a hydroxyl group, which includes an acetyl group, a tetrahydro-4H-pyran-2-yl group, a tri(alkyl/aryl)silyl group such as a trimethylsilyl group, a triethylsilyl group, a t-butyldimethylsilyl group or a t-butyldiphenylsilyl group, or the like].

The compounds expressed by the above-mentioned formulae (5) or (6) can be obtained from vitamin D₂ by the method described in Tetrahedron, 20, 4609-4619 (1987).

The conversion from the above formula (7) to the above formula (8), and that from the above formula (9) to the above formula (10) can be achieved through photoisomerization by the same method as the conversion from the above formula (5) to the above formula (6).

The conversion from the above formula (6) to the above formula (8) can be carried out as shown below. That is, a compound whose A in the above formula (8) is -CH=CH-, -C≡C- or -CH=CH-CH=CH-, and a compound whose A in the above formula (8) is -CH₂-, -CH₂-CH=CH-, or -CH2-C≡C- can be obtained by combining known methods as shown in the following scheme 7 and scheme 8, respectively.

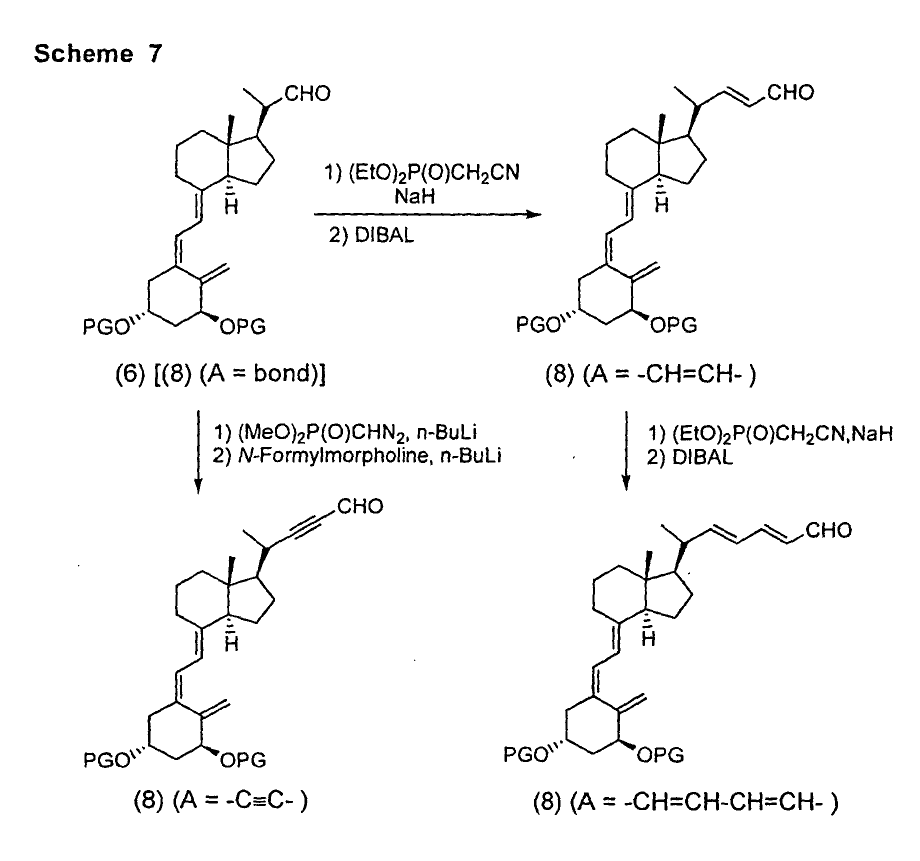

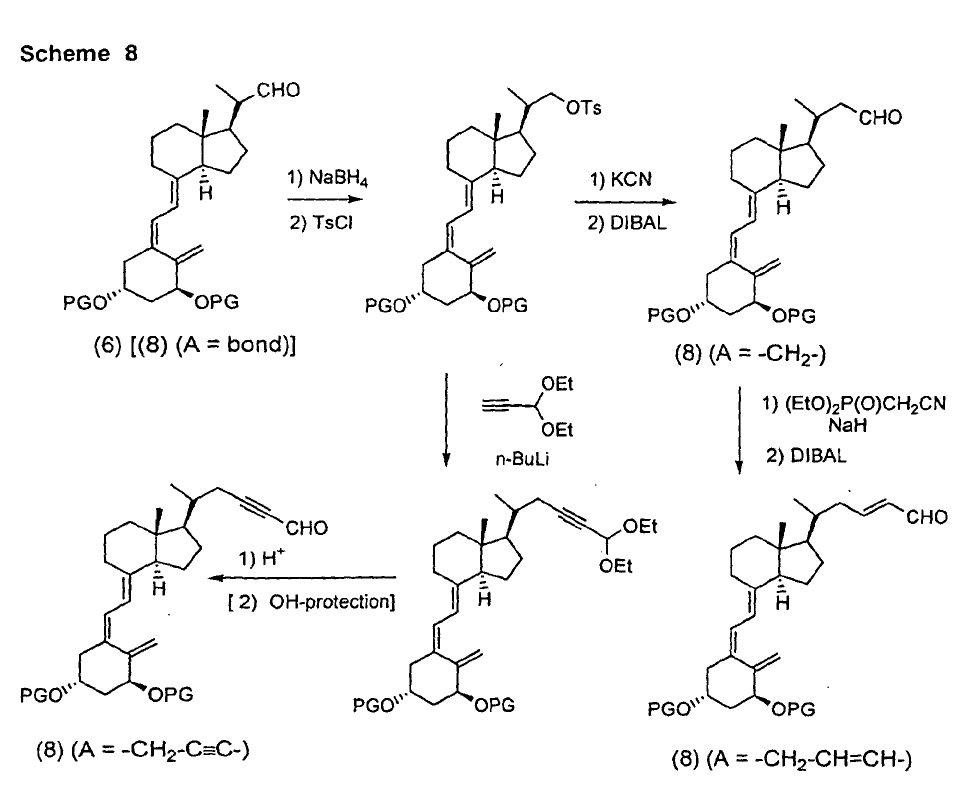

The conversion from the above formula (5) to the above formula (7) can be conducted in a same manner as the conversion from the above formula (6) to the above formula (8) shown above.

The conversions from the above formula (7) to the above formula (9), from the above formula (8) to the above formula (10) and from the above formula (10) to the above formula (1) can be conducted in the same manner as shown in the above-mentioned scheme 1.

A vitamin D₃ derivative obtained through the above-mentioned processes can be converted into a pharmaceutically permissible solvate shown above at need.

Further, the present invention provides treating agents containing a vitamin D₃ derivative shown by the above formula (1) or a pharmaceutically permissible solvate thereof for osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia or Paget's disease of bone. Out of these diseases, hyperparathyroidism and Paget's disease of bone are preferably cited as objective diseases of the present invention.

The treating agent of the present invention can be administered orally, or parentally such as intravenously, subcutaneously, intramuscularly, percutaneously, intranasally, intrarectally or the like, or by inhalation. Dosage forms for oral administration include tablets, pills, powders, granules, liquids, suspensions, syrups, capsules, etc.

The tablets are formulated according to a conventional process by using additives consisting of an excipient such as lactose, starch, calcium carbonate, crystalline cellulose or silicic acid; a binder such as carboxymethylcellulose, methylcellulose, calcium phosphate or polyvinylpyrrolidone; a disintegrator such as sodium alginate, sodium hydrogencarbonate, sodium laurylsulfate or stearic acid monoglyceride; a humectant such as glycerin; an absorbent such as kaolin or colloidal silica; a lubricant such as talc or granular boric acid; etc.

The pills, powders and granules are prepared by conventional processes also using additives similar to those mentioned above.

Liquid preparations such as the liquids, suspensions and syrups can be formulated also according to conventional processes. As a support, for example, a glycerol ester such as tricaprylin, triacetin or an iodized poppy oil fatty acid ester; water; an alcohol such as ethanol; or an oily base such as liquid paraffin, coconut oil, soybean oil, sesame oil or corn oil is used.

The capsules are formulated by filling a pharmaceutical composition such as powder, granule or liquid in gelatin capsules, or the like.

Dosage forms for intravenous, subcutaneous and intramuscular administration include injections in a form of a sterilized aqueous or non-aqueous solution, or the like. In an aqueous solution, for example, a physiological saline solution or the like is used as a solvent. In a non-aqueous solution, for example, propylene glycol, polyethylene glycol, a vegetable oil such as olive oil, an organic ester which is acceptable for injection such as ethyl oleate or an iodized poppy oil fatty acid ester, or the like is used as a solvent. To the pharmaceutical preparations for injection are optionally added an isotonizing agent, a disinfectant, a humectant, an emulsifier, a dispersant, a stabilizer, etc., and the preparation may be sterilized by applying an adequate treatment such as filtration through a bacterium-retaining filter, blending of a germicide or irradiation. Also, an aseptic solid preparation is produced, and it is used for injection by dissolving in sterilized water or a sterilized solvent for injection just prior to use. Further, a compound of the present invention may be used in the form of a clathrate compound prepared by using α, β or γ-cyclodextrin, a methylated cyclodextrin, or the like. The compound may be administered also in an injection of a lipoid form.

Dosage forms for percutaneous administration include ointments, creams, lotions, solutions, etc. Examples of the base of an ointment include a fatty oil such as castor oil, olive oil, sesame oil or safflower oil; lanolin; white, yellow or hydrophilic vaseline; wax; a higher alcohol such as oleyl alcohol, isostearyl alcohol, octyldodecanol or hexyldecanol; a glycol such as glycerin, diglycerin, ethylene glycol, propylene glycol, sorbitol or 1,3-butanediol; etc. Further, as a solubilizing agent for a compound of the present invention, ethanol, dimethyl sulfoxide, polyethylene glycol, etc., may be compounded. Optionally, a preservative such as a paraoxybenzoic acid ester, sodium benzoate, salicylic acid, sorbic acid or boric acid; an antioxidant such as butylhydroxyanisole or dibutylhydroxytoluene; etc., may be added. Further, in order to stimulate percutaneous absorption, an absorption promoter such as diisopropyl adipate, diethyl sebacate, ethyl caproate or ethyl laurate may be compounded. Also, for stabilization, a compound of the present invention may be used in the form of a clathrate compound prepared by using α, β or γ-cyclodextrin, a methylated cyclodextrin, etc.

An ointment can be prepared by a conventional process. In the creams, O/W-type dosage forms are preferable for stabilizing compounds of the present invention. Further, the above-mentioned fatty oil, higher alcohol, glycol or the like may be used as the base of a cream, and an emulsifier such as diethylene glycol, propylene glycol, sorbitan mono fatty acid ester, polysorbate 80 or sodium laurylsulfate may be used. Further, the above-mentioned preservative, antioxidant or the like may be added, as necessary. Furthermore, similarly to the case of ointment, a compound of the present invention can be used in the form of a clathrate compound prepared by using a cyclodextrin or a methylcyclodextrin. A cream can be prepared according to a conventional process.

Examples of the lotions include a suspension-type lotion, an emulsion-type lotion and a solution-type lotion. The suspension-type lotion is prepared by using a suspending agent such as sodium alginate, traganth or sodium carboxymethylcellulose, and optionally by adding an antioxidant, a preservative, etc. The emulsion-type lotion is prepared according to a conventional process by using an emulsifier such as sorbitan mono fatty acid ester, polysorbate 80 or sodium laurylsulfate. As the solution-type lotion, a compound of the present invention is dissolved in an alcohol such as ethanol, optionally adding an antioxidant, a preservative or the like.

Pastas, poultices, aerosols, etc., may be cited besides the above-mentioned dosage forms. These pharmaceutical preparations can be prepared according to conventional processes.

Pharmaceutical preparations for intranasal administration are supplied in the form of a liquid or powdery composition. As the base of the liquid preparation, water, saline, a phosphate buffer solution, an acetate buffer solution, or the like is used, and the liquid preparation may contain further a surfactant, an antioxidant, a stabilizer, a preservative and/or a thickener. As the base for the powdery preparation, a water-absorbent base is preferable. Examples of the water-absorbent base include polyacrylate salts such as sodium polyacrylate, potassium polyacrylate and ammonium polyacrylate; cellulose lower-alkyl ethers such as methylcellulose, hydroxyethylcellulose, hydroxypropyl-cellulose and sodium carboxymethylcellulose; and polyethylene glycol, polyvinyl pyrrolidone, amylose, pullulan, etc., which are easily soluble in water. Further, they include cellulose compounds such as crystalline cellulose, α-cellulose and cross-linked sodium carboxymethylcellulose; starch compounds such as hydroxypropyl starch, carboxymethyl starch, cross-linked starches, amylose, amylopectin and pectin; proteins such as gelatin, casein and sodium caseinate; gums such as gum arabic, tragacanth gum and glucomannan; and polyvinylpolypyrrolidone, cross-linked polyacrylic acid and salts thereof, cross-linked polyvinyl alcohols, etc., which are scarcely soluble in water. These compounds may be used in a mixture thereof. The powdery preparation may be further compounded with an antioxidant, a coloring agent, a preservative, a disinfectant, an antiseptic, etc. These liquid and powdery preparations can be administered, for example, by using a spraying device, etc.

For intrarectal administration, ordinary suppositories such as gelatin soft capsule are used.

Further, for inhalation, a powdery or liquid composition prepared by using an active ingredient of a vitamin D₃ derivative of the present invention alone or in combination with an adequate biocompatible vehicle can be administered to disease sites using an applicator such as a spraying device, a nebulizer or an atomizer. Alternatively, an active ingredient may be administered to disease sites by suspending the active ingredient in a spraying agent for aerosol such as flon.

A pharmaceutically effective dose of an active ingredient of the present invention depends on an administration route, the age and sex of the patient, and the conditions of the disease, but it is ordinarily about 0.001-10000 µg per day, and administration frequency is ordinarily from 1-3 times per day to 1-3 times per week. The pharmaceutical preparation is preferably prepared so as to meet these conditions.

Further, a treating agent of the present invention can be administered in combination with a conventional medicine.

Effectiveness of a vitamin D₃ derivative expressed by the above formula (1) of the present invention as a treating agent for osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia or Paget's disease of bone was expressed by the parameter consisting of the binding capacity of the compound of the present invention to a 1α, 25-dihydroxyvitamin D₃ receptor (VDR), and this was concretely shown in an example mentioned below. That is, it has become clear that the compound of the present invention binds to VDR with extremely high affinity. The pharmacological activity of the vitamin D₃ derivatives is exerted through VDR. Therefore compounds having high binding capacity to VDR are useful as treating agents for the above-mentioned diseases.

Further, usefulness of a vitamin D₃ derivative expressed by the above formula (1) of the present invention for treating hypercalcemia and Paget's disease of bone was exhibited with a parameter consisting of differentiation-inducing effect determined by using HL-60 cell, and this was concretely shown in an example mentioned below. That is, the compound of the present invention suppresses specifically the differentiation of HL-60 cell which has been induced by 1α, 25-dihydroxyvitamin D₃, and this has revealed that the compound of the present invention acts as a vitamin D₃ antagonist. Since hypercalcemia and Paget's disease of bone are induced as a result of the sthenia in the activity of active vitamin D₃, the vitamin D₃ antagonist is useful as a treating agent for these diseases.

The present invention will be explained further in detail henceforth with examples, while the present invention is not restricted by the examples. The compound numbers in every example correspond to the compound numbers shown in Table 1. A compound whose compound number has an alphabet shows a stereoisomer of the compound having the corresponding bare compound number.

### Examples

### Example 1

### Production of 20(R)-(tetrahydro-3-vinylidene-2-furanon-5-yl)-9,10-secopregna-5(Z),7(E),10( 19)-triene-1(S),3(R)-diol (Compound No. 11a and Compound No. 11b)

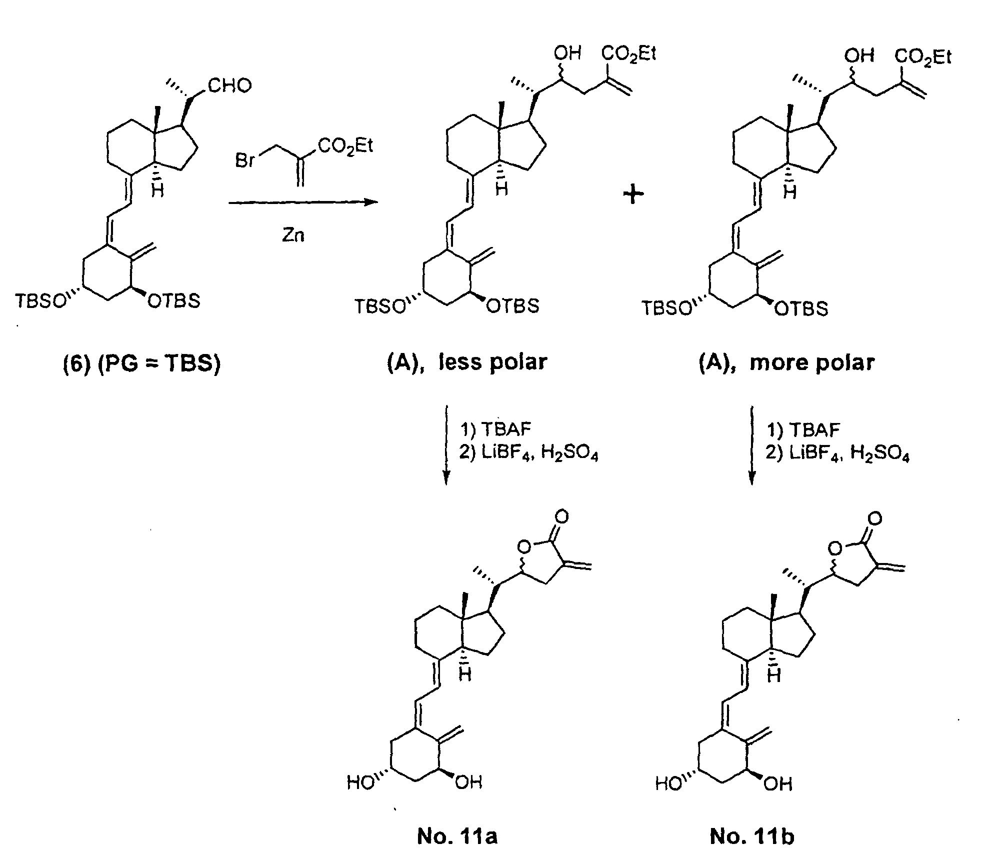

(1) Eighty-six mg (0.15 mmol) of Compound (6) (PG=TBS), which can be obtained by a known method (Tetrahedron, 20, 4609-4619, (1987)) was dissolved in dehydrated THF (3 ml), and the solution was ice-cooled. To the solution were added 15 mg (0.23 mmol) of zinc (powder) and 43 mg (0.23 mmol) of ethyl 2-(bromomethyl)acrylate, lastly 0.2 ml of a saturated ammonium chloride aqueous solution was added, and the mixture was stirred for 10 min under ice cooling and for 1.5 hr at room temperature. The reaction mixture was extracted with ethyl acetate after the addition of a saturated ammonium chloride aqueous solution. The organic layer was washed with brine, dried and concentrated. The residue was purified by silica gel column chromatography (hexane : ethyl acetate=20:1-15:1) and preparative TLC (hexane : ethyl acetate=4:1) to obtain Compound (A) of the lower polarity body (12 mg, 12% yield) and Compound (A) of the higher polarity body (31 mg, 30% yield). They were colorless oil. Compound (A) of the lower polarity body and Compound (A) of the higher polarity body are diastereoisomers based on the asymmetric carbon to which the hydroxyl group formed by the present reaction is bonded.
Compound (A) of the lower polarity body:
¹H NMR (CDCl₃) δ : 0.06 (s, 12 H), 0.56 (s, 3 H), 0.88 (s, 18 H), 0.98 (d, J=6.8 Hz, 3 H), 1.25-2.55 (m, 21 H), 2.81-2.85 (m, 1 H), 3.76-3.80 (m, 1 H), 4.11-4.27 (m, 4 H), 4.35-4.37 (m, 1 H), 4.86 (br., 1 H), 5.18 (s, 1 H), 5.65-5.88 (m, 1 H), 6.03 (d, J=11.2 Hz, 1 H), 6.22-6.26 (m, 2 H).
MS m/z 687.5 (M+1)⁺.
Compound (A) of the higher polarity body:
¹H NMR (CDCl₃) δ : 0.06 (s, 12 H), 0.54 (s, 3 H), 0.88 (s, 18 H), 0.97 (d, J=6.8 Hz, 3 H), 1.23-2.54 (m, 21 H), 2.81-2.85 (m, 1 H), 3.81-3.84 (m, 1 H), 4.11-4.27 (m, 4 H), 4.37 (br., 1 H), 4.86 (d, J=2.1 Hz, 1 H), 5.17 (d, J=1.7 Hz, 1 H), 5.65 (s, 1 H), 6.02 (d, J=11.5 Hz, 1 H), 6.23-6.26 (m, 2 H).
MS m/z 687.5 (M+1)⁺

(2-1) Compound (A) (12 mg, 17.5 µmol) of the lower polarity body obtained in the above process was dissolved in anhydrous THF (2 ml), and the solution was ice-cooled. To the solution was added 26 µl (1N, 26 µmol) of a THF solution of TBAF, and the mixture was stirred for 1 hr under ice cooling. Further, 26 µl (1N, 26 µmol) of a THF solution of TBAF was added, and the mixture was stirred for 1 hr. The reaction mixture was extracted with ethyl acetate after the addition of water. The organic layer was washed with brine, dried and concentrated to obtain a lactone ring body.
The lactone ring body:
¹H NMR (CDCl₃) δ : 0.06 (s, 12 H), 0.58 (s, 3 H), 0.88 (s, 18 H), 0.89-2.42 (m, 20 H), 2.77-2.87 (m, 2 H), 4.19 (br., 1 H), 4.37 (br., 1 H), 4.64-4.65 (m, 1 H), 4.85 (s, 1 H), 5.18 (s, 1 H), 5.61 (s, 1 H), 6.03 (d, J=11.1 Hz, 1 H), 6.22-6.56 (m, 2 H).
MS m/z 641.5 (M+1)⁺.

The obtained lactone body was dissolved in a mixed solution of acetonitrile (1.5 ml) and methylene chloride (1.5 ml), 5 mg of LiBF₄ (52 µmol) was added to the solution, and the solution was ice-cooled. To the solution was added 31 µl (1 N, 31 µmol) of an acetonitrile solution of sulfuric acid solution, and the mixture was stirred for 45 min under ice cooling. The reaction mixture was extracted with ethyl acetate after the addition of a saturated sodium hydrogencarbonate aqueous solution. The organic layer was washed with brine, dried and concentrated. The residue was purified by HPLC (reversed phase, elution: A; 95% H₂O/CH₃CN, B; 95% MeOH/H₂O, B=76%) to obtain the objective Compound No. 11a (2.5 mg, 35% yield) of white solid in purity of 99.1%.
Compound No. 11a:
¹H NMR (CDCl₃) δ : 0.60 (s, 3 H), 0.88 (d, J=6.6 Hz, 3 H), 1.23-2.14 (m, 16 H), 2.32 (dd, J=6.3 and 13.0 Hz, 1 H), 2.58-2.62 (m, 1 H), 2.75-2.86 (m, 3 H), 4.24 (br., 1 H), 4.43 (br., 1 H), 4.64 (dt, J=4.0 and 7.1 Hz, 1H), 5.00 (s, 1 H), 5.33 (d, J=1.5 Hz, 1 H), 5.61 (t, J=2.6 Hz, 1 H), 6.03 (d, J=11.2 Hz, 1 H), 6.22 (t, J=2.6 Hz, 1 H), 6.37 (d, J=11.4 Hz, 1 H).
MS m/z 413.2 (M+1)⁺.

(2-2) Compound (A) of the higher polarity (39 mg, 56.8 µmol) obtained above was dissolved in 3 ml of anhydrous THF, and the solution was ice-cooled. To the solution was added 170 µl (1 N, 170 µmol) of a THF solution of TBAF, the mixture was stirred for 1 hr under ice cooling, and further for 1 hr after 170 µl (1 N, 170 µmol) of a THF solution of TBAF was added. The reaction mixture was extracted with ethyl acetate after the addition of water, and the organic layer was washed with brine, dried and concentrated to obtain a lactone ring body.
The lactone ring body:
¹H NMR (CDCl₃) δ : 0.06 (s, 12 H), 0.54 (s, 3 H), 0.88 (s, 18 H), 0.89-3.02 (m, 22 H), 4.19 (br., 1 H), 4.38 (br., 1 H), 4.7 3(t, J=7.1 Hz, 1 H), 4.86 (d, J=2.1 Hz, 1 H), 5.18 (d, J=1.3 Hz, 1 H), 5.62 (s, 1 H),6.02 (d, J=11.2 Hz, 1 H), 6.22-6.56 (m, 2 H).
MS m/z 641.5 (M+1)⁺.

The obtained lactone body was dissolved in a mixed solvent of acetonitrile (2 ml) and methylene chloride (2 ml), and to the solution was added 16 mg (170 µmol) of LiBF₄, and the solution was ice-cooled. To the solution was added 102 µl (1N, 120 µmol) of an acetonitrile solution of sulfuric acid, and the mixture was stirred for 45 min under ice cooling. The reaction mixture was extracted with ethyl acetate after the addition of a saturated sodium hydrogencarbonate aqueous solution. The organic layer was washed with brine, dried and concentrated. The residue was purified by HPLC (reversed phase, elution: A; 95% H₂O/CH₃CN, B; 95% MeOH/H₂O, B=76%) to obtain the objective Compound No. 11b (12.3 mg, 53% yield) of white solid in purity of 99.0%.
Compound No. 11b:
¹H NMR (CDCl₃) δ : 0.06 (s, 3 H), 0.91 (d, J=6.6 Hz, 3 H), 1.32-1.83 (m, 11 H), 1.93-2.09 (m, 5 H), 2.31 (dd, J=6.8 and 13.4 Hz, 1 H), 2.57-2.75 (m, 2 H), 2.80-2.86 (m, 1 H), 2.92-3.02 (m, 1 H), 4.22 (br., 1 H), 4.43 (br., 1 H), 4.69-4.74 (m, 1 H), 5.00 (s, 1 H), 5.33 (s, 1 H), 5.62 (t, J=2.5 Hz, 1 H), 6.02 (d, J=11.4 Hz, 1 H), 6.22 (t, J=3.0 Hz, 1 H), 6.38 (d, J=11.2 Hz, 1 H).
MS m/z 413.2 (M+1)⁺.

### Example 2

### Production of 20(R)-(2-(tetrahydro-3-vinylidene-2-furanon-5-yl)-1(E)-ethenyl)-9,10-secopregna-5(Z),7(E),10(19)-triene-1(S),3(R)-diol (Compound No. 31a and Compound No. 31b)

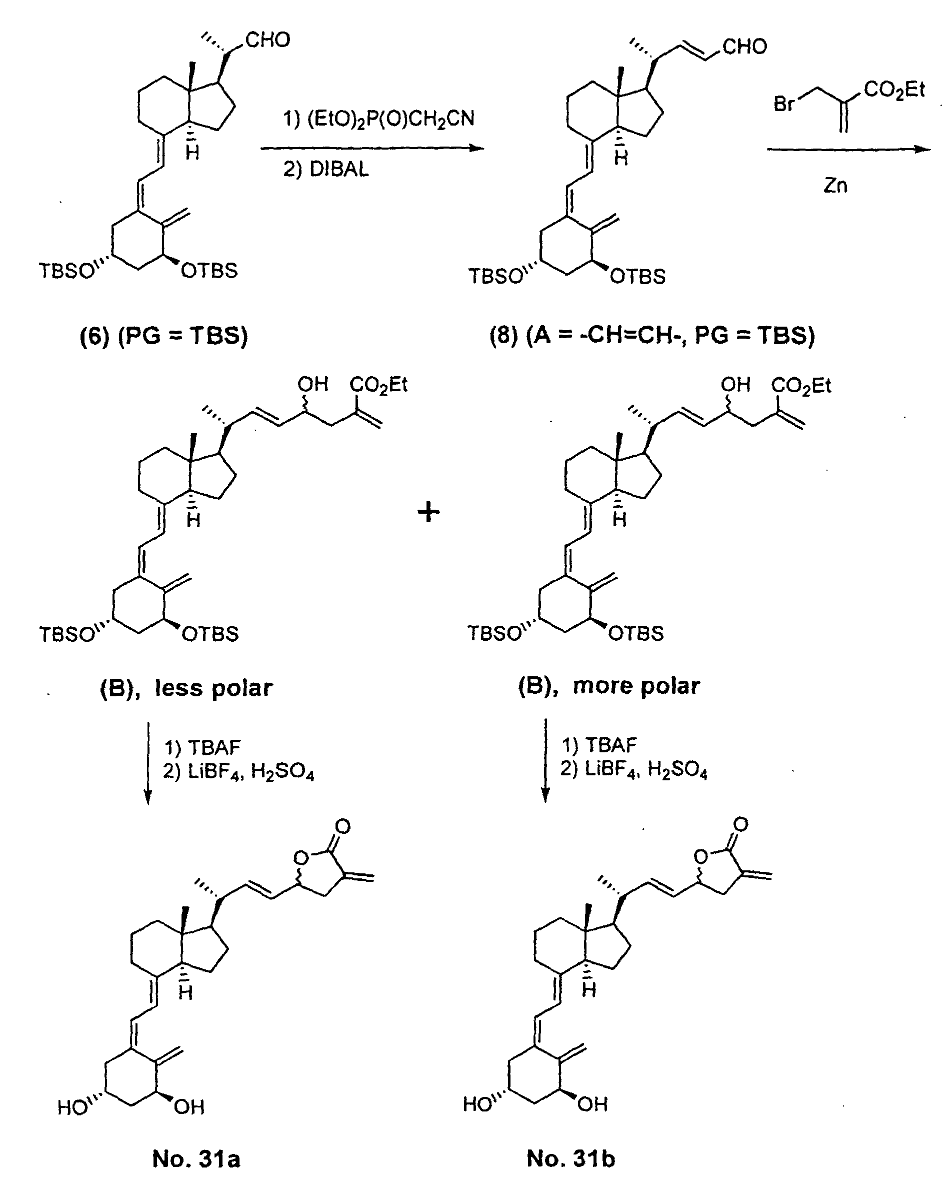

(1) Under nitrogen atmosphere, 88 mg (2.2 mmol) of sodium hydride was dissolved in 15 ml of anhydrous THF, and the solution was ice-cooled. To the solution was added 425 mg (2.4 mmol) of diethyl cyanomethylphosphonate, and the mixture was stirred for 40 min under ice cooling. To the solution, an anhydrous THF (5 ml) solution of 1.15 g (2.0 mmol) of Compound (6) (PG=TBS), which had been manufactured through a known method (Tetrahedron, 20, 4609-4619, (1987)), was added dropwise over 5 min, and successively the mixture was stirred for 40 min under ice cooling. The reaction mixture was extracted with ethyl acetate after the addition of a saturated ammonium chloride aqueous solution. The organic layer was washed with brine, dried and concentrated to obtain a crude Wittig adduct (1.29 g). The obtained crude Wittig adduct was dissolved in 10 ml of anhydrous methylene chloride under a nitrogen atmosphere, and the solution was cooled to -70°C. A toluene solution (2.97 ml, 1.01 M, 3.0 mmol) of DIBAL was added dropwise to the above solution, and the mixture was stirred for 2 hr at this temperature. To the reaction mixture were added water (10 ml) and a saturated sodium sulfate aqueous solution (10 ml), the mixture was stirred for 10 min at room temperature, and the reaction mixture was extracted with twice with methylene chloride after the addition of 1 N hydrochloric acid. The organic layer was washed with a saturated sodium hydrogencarbonate aqueous solution and then with brine, dried and concentrated. The residue was purified by silica gel column chromatography (hexane : ethyl acetate=80:1 to 20:1) to obtain the objective product (8) (A=-CH=CH- and PG=TBS) (598 mg, 50% yield) of a white foamy product.
Compound (8) (A=-CH=CH- and PG=TBS):
¹H NMR (CDCl₃) δ : 0.06 (s, 6 H), 0.06 (s, 6 H), 0.59 (s, 3 H), 0.88 (s, 18 H), 1.14-1.86 (m, 14 H), 1.97-2.01 (m, 2 H), 2.18-2.26 (m, 1 H), 2.42-2.47 (m, 2 H), 2.82-2.86 (m, 1 H), 4.17 (m, 1 H), 4.36 (m, 1 H), 4.85 (d, J=2.3 Hz, 1 H), 5.18 (d, J=2.5 Hz, 1 H), 6.00-6.10 (m, 2 H), 6.23 (d, J=10.9 Hz, 1 H), 6.72 (dd, J=8.7 and 15.5 Hz, 1 H), 9.49 (d, J=7.9 Hz, 1 H).
MS m/z 599.5 (M+1)⁺.

(2) Compound (8) (A=-CH=CH- and PG=TBS) (93 mg, 0.155 mmol) obtained above was dissolved in an anhydrous THF solution (3 ml), and the solution was ice-cooled. To the solution were added zinc powder (15 mg, 0.23 mmol) and ethyl 2-(bromomethyl)acrylate (45 mg, 0.23 mmol), and lastly 0.2 ml of a saturated ammonium chloride aqueous solution, and the mixture was stirred for 5 min under ice cooling and for 1 hr at room temperature. The reaction mixture was extracted with ethyl acetate after the addition of a saturated ammonium chloride aqueous solution. The organic layer was washed with brine, dried and concentrated, and the residue was purified by silica gel column chromatography (hexane : ethyl acetate=40:1-15:1) and preparative TLC (hexane : ethyl acetate=3:1) to obtain Compound (B) of the lower polarity body (39 mg, 35% yield) and Compound (B) of the higher polarity body (42 mg, 38% yield). They were colorless oil. Compound (B) of the lower polarity body and Compound (B) of the higher polarity body are diastereoisomers based on the asymmetric carbon to which the hydroxyl group formed by the present reaction is bonded.
Compound (B) of the lower polarity body:
¹H NMR (CDCl₃) δ : 0.06 (s, 12 H), 0.54 (s, 3 H), 0.88 (s, 18 H), 1.02 (d, J=6.6 Hz, 3 H), 1.23-2.25 (m, 19 H), 2.43-2.60 (m, 3 H), 2.82 (d, J=10.0 Hz, 1 H), 4.11-4.26 (m, 4 H), 4.37 (br., 1 H), 4.86 (d, J=2.3 Hz, 1 H), 5.18 (d, J=1.5 Hz, 1 H), 5.39 (dd, J=6.3 and 15.3 Hz, 1 H), 5.53 (dd, J=8.1 and 15.3 Hz, 1 H), 5.64 (d, J=1.3 Hz, 1 H), 6.01 (d, J=11.2 Hz, 1 H), 6.23 (d, J=10.7 Hz, 1 H), 6.25 (s, 1 H).
MS m/z 713.5 (M+1)⁺.
Compound (B) of the higher polarity body:
¹H NMR (CDCl₃) δ : 0.06 (s, 12 H), 0.54 (s, 3 H), 0.88 (s, 18 H), 1.03 (d, J=6.6 Hz, 3 H), 1.23-2.25 (m, 19 H), 2.43-2.62 (m, 3 H), 2.82 (d, J=9.9 Hz, 1 H), 4.11-4.26 (m, 4 H), 4.36 (br., 1 H), 4.86 (d, J=2.5 Hz, 1 H), 5.18 (d, J=1.5 Hz, 1 H), 5.38 (dd, J=6.6 and 15.3 Hz, 1 H), 5.51 (dd, J=8.2 and 15.3 Hz, 1 H), 5.65 (s, 1 H), 6.01 (d, J=11.2 Hz, 1 H), 6.23 (d, J=11.4 Hz, 1 H), 6.25 (s, 1 H).
MS m/z 713.5 (M+1)⁺.

(3-1) Compound (B) (39 mg, 54.7 µmol) of the lower polarity body obtained above was dissolved in 3 ml of an anhydrous THF, and the solution was ice-cooled. To the solution was added a THF solution (55 µl, 1 N, 55 µmol) of TBAF, the mixture was stirred for 30 min under ice cooling, and further the mixture was stirred for 30 min under ice cooling after the addition of a THF solution (55 µl, 1 N, 55 µmol) of TBAF. The reaction mixture was extracted with ethyl acetate after the addition of water. The organic layer was washed with brine, dried and concentrated to obtain a lactone ring body.
Lactone ring body:
¹H NMR (CDCl₃) δ : 0.06 (s, 12 H), 0.55 (s, 3 H), 0.88 (s, 18 H), 0.92-3.39 (m, 22 H), 4.19 (br., 1 H), 4.38 (br., 1H), 4.85-4.92 (m, 2 H), 5.18 (s, 1H), 5.41 (dd, J=7.3 and 15.2 Hz, 1 H), 5.63 (s, 1 H), 5.68 (dd, J=8.6 and 15.5 Hz, 1 H), 6.01 (d, J=11.2 Hz, 1 H), 6.21-6.25 (m, 2 H).
MS m/z 667.5 (M+1)⁺.

The obtained lactone body was dissolved in a mixed solvent of acetonitrile (2 ml) and methylene chloride (2 ml), to the solution was added LiBF₄ (15 mg, 164 µmol), and the mixture was ice-cooled. An acetonitrile solution (100 µl, 1 N, 100 µmol) of sulfuric acid was added to the solution, and the mixture was stirred for 45 min under ice cooling. The reaction mixture was extracted with ethyl acetate after the addition of a saturated sodium hydrogencarbonate aqueous solution. The organic layer was washed with brine, dried and concentrated. The residue was purified by HPLC (reversed phase, elution: A; 95% H₂O/CH₃CN, B; 95% MeOH/H₂O, B=81%) to obtain the objective Compound No. 31a (10.2 mg, 43% yield) of white solid in purity of 98.7%.
Compound No. 31a:
¹H NMR (CDCl₃) δ : 0.56 (s, 3 H), 1.04 (d, J=6.6 Hz, 3 H), 1.26-2.17 (m, 16 H), 2.31 (dd, J=6.8 and 13.2 Hz, 1 H), 2.58-2.72 (m, 2 H), 2.80-2.85 (m, 1 H), 3.11 (ddt, J=2.5, 7.8 and 17.0 Hz, 1 H), 4.23 (br., 1 H), 4.43 (br., 1 H), 4.88 (q, J=6.8 Hz, 1 H), 5.00 (s, 1 H), 5.33 (s, 1 H), 5.41 (dd, J=7.3 and 15.3 Hz, 1 H), 5.63 (t, J=2.3 Hz, 1 H), 5.68 (dd, J=8.9 and 15.5 Hz, 1 H), 6.01 (d, J=11.2 Hz, 1 H), 6.23 (t, J=2.8 Hz, 1 H), 6.37 (d, J=11.4 Hz, 1 H).
MS m/z 439.2 (M+1)⁺.

(3-2) Compound (B) (42 mg, 58.9 µmol) of the higher polarity body obtained above was dissolved in 3 ml of an anhydrous THF, and the solution was ice-cooled. To the solution was added a THF solution (59 µl, 1 N, 59 µmol) of TBAF, and the mixture was stirred for 30 min under ice cooling. Subsequently, a THF solution (59 µl, 1 N, 59 µmol) of TBAF was further added, and the mixture was stirred for 30 min under ice cooling. The reaction mixture was extracted with ethyl acetate after the addition of water. The organic layer was washed with brine, dried and concentrated to obtain a lactone ring body.
The lactone ring body:
¹H NMR (CDCl₃) δ : 0.06 (s, 12 H), 0.55 (s, 3 H), 0.88 (s, 18 H), 0.91-3.39 (m, 22 H), 4.19 (br., 1 H), 4.37 (br., 1 H), 4.85-4.92 (m, 2 H), 5.18 (s, 1 H), 5.41 (dd, J=7.3 and 15.3 Hz, 1 H), 5.61-5.63 (m, 1 H), 5.67 (dd, J=8.3 and 15.3 Hz, 1 H), 6.01 (d, J=11.4 Hz, 1 H), 6.21-6.25 (m, 2 H).
MS m/z 667.5 (M+1)⁺.

The obtained lactone body was dissolved in a mixed solvent of acetonitrile (2 ml) and methylene chloride ( 2 ml), to the solution was added LiBF₄ (17 mg, 0.18 mmol), and the solution was ice-cooled. To the solution was added an acetonitrile solution (106 µl, 1 N, 106 µmol) of sulfuric acid, and the mixture was stirred for 45 min under ice cooling. The reaction mixture was extracted with ethyl acetate after the addition of a saturated sodium hydrogencarbonate aqueous solution. The organic layer was washed with brine, dried and concentrated. The residue was purified by HPLC (reversed phase, elution: A; 95% H₂O/CH₃CN, B; 95% MeOH/H₂O, B=81%) to obtain the objective Compound No. 31b (9.6 mg, 37% yield) of colorless film in purity of 99.3%.
Compound No. 31b:
¹H NMR (CDCl₃) δ : 0.56 (s, 3 H), 1.06 (d, J=6.8 Hz, 3 H), 1.21-2.14 (m, 16 H), 2.32 (dd, J=6.3 and 13.7 Hz, 1 H), 2.58-2.72 (m, 2 H), 2.80-2.86 (m, 1 H), 3.11 (ddt, J=2.5, 7.8 and 17.0 Hz, 1 H), 4.23 (br., 1 H), 4.43 (br., 1 H), 4.89 (q, J=6.8 Hz, 1 H), 5.00 (s, 1 H), 5.33 (s, 1 H), 5.41 (dd, J=7.3 and 15.3 Hz, 1 H), 5.63 (t, J=2.5 Hz, 1 H), 5.68 (dd, J=8.5 and 15.0 Hz, 1 H), 6.01 (d, J=11.5 Hz, 1 H), 6.23 (t, J=2.8 Hz, 1 H), 6.37 (d, J=11.2 Hz, 1 H).
MS m/z 439.2 (M+1)⁺.

### Example 3

### Production of 20(R)-(2-(tetrahydro-3-vinylidene-2-furanon-5-yl)-1(E),3(E)-butadienyl)-9,10-secopregna-5(Z),7(E),10(19)-triene-1(S),3(R)-diol (Compound No. 51a and Compound No. 51b)

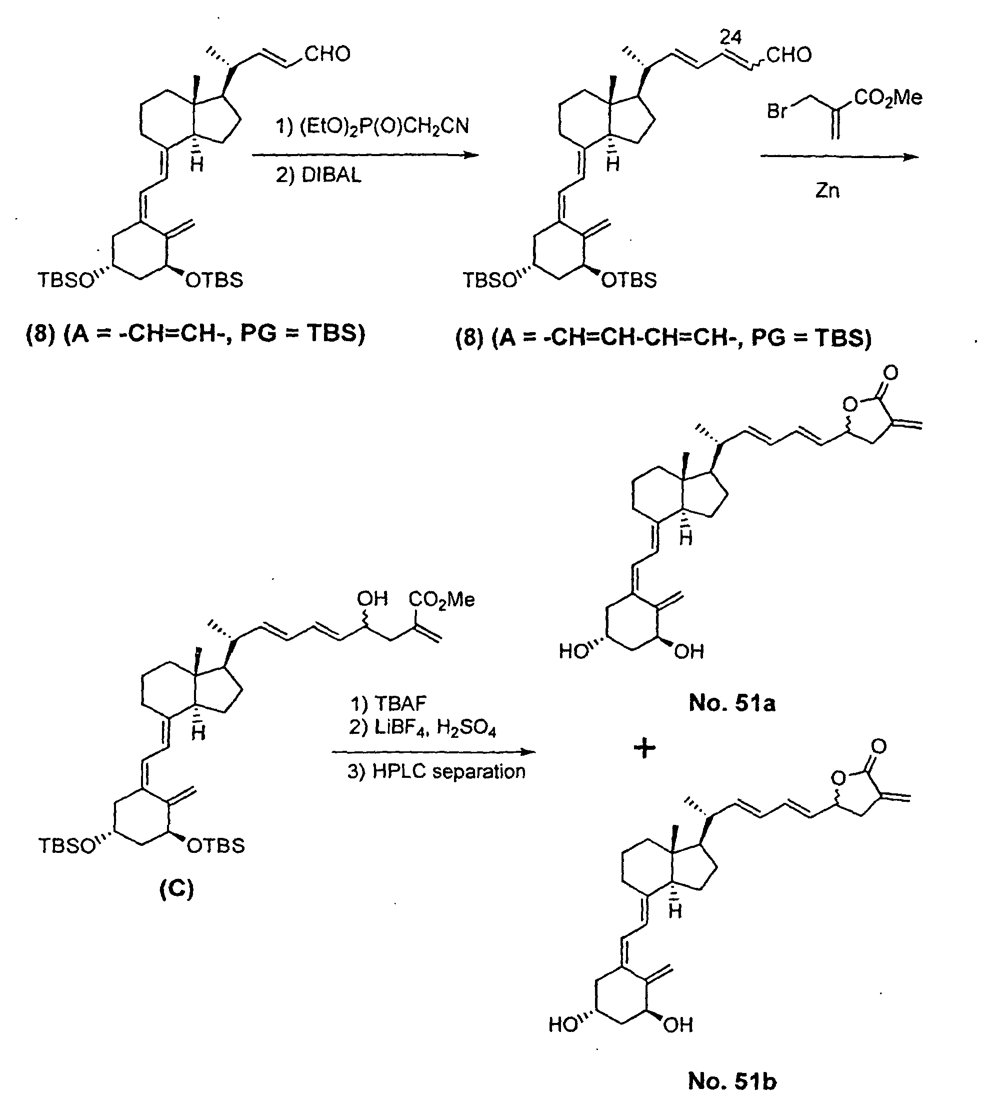

(1) To an anhydrous THF solution (1 ml) of sodium hydride (10 mg, 0.24 mmol) was added diethyl cyanomethylphosphonate (38 µl, 0.36 mmol), and the mixture was stirred for 15 min at 0°C. To the solution was added dropwise an anhydrous THF solution (2 ml) of Compound (8) (A=-CH=CH-, PG=TBS) (95 mg, 0.159 mmol) obtained in Example 2 (1), and the mixture was stirred for 10 min at 0°C. The reaction was quenched with a saturated ammonium chloride aqueous solution, and the mixture was extracted with ethyl acetate. The organic layer was washed with water and then brine, dried over anhydrous magnesium sulfate and concentrated. To the methylene chloride solution (1 ml) of the residue was added dropwise 0.31 ml of a toluene solution of DIBAL at -78°C over 1 hr, 0.47 ml of a toluene solution of DIBAL was further added, and the mixture was stirred for 10 min. The temperature was gradually raised (-78°C →-30°C) over the time from the first addition of DIBAL to the completion of the reaction. The reaction was quenched with an aqueous solution of citric acid, and the mixture was extracted with ethyl acetate. The organic layer was washed with water and then brine, dried over anhydrous magnesium sulfate, and concentrated. The residue was purified by column chromatography (hexane : ethyl acetate=60:1 to 20:1) to obtain Compound (8) (A=-CH=CH-CH=CH-, PG=TBS, 24(E)) (50 mg, 50% yield) and Compound (8) (A=-CH=CH-CH=CH-, PG=TBS, 24(Z)) (13 mg, 13% yield).
Compound (8) (A=-CH=CH-CH=CH-, PG=TBS, 24(E)):
¹H NMR (CDCl₃) δ : 0.03 (s, 6 H), 0.05 (s, 6 H), 0.57 (s, 3 H), 0.88 (s, 18 H), 1.11 (d, J=6.6 Hz, 3 H), 1.23-2.02 (m, 13 H), 2.18-2.35 (m, 2 H), 2.42-2.47 (m, 1 H), 2.80-2.87 (m, 1 H), 4.15-4.23 (m, 1 H), 4.85 (d, J=2.2 Hz, 1 H), 5,17 (s, 1 H), 6.02 (d, J=11.0 Hz, 1 H), 6.07 (dd, J=8.1 and 15.2 Hz, 1 H), 6.14 (dd, J=8.8 and 14.9 Hz, 1 H), 6.23 (d, J=11.0 Hz, 1 H), 6.26 (dd, J=10.5 and 14.9 Hz, 1 H), 7.07 (dd, J=10.5 and 15.2 Hz, 1 H), 9.53 (d, J=8.1 Hz, 1 H).
MS m/z 625 (M+1)⁺.
Compound (8) (A=-CH=CH-CH=CH-, PG=TBS, 24(Z)):
¹H NMR (CDCl₃) δ : 0.055 (s, 6 H), 0.063 (s, 6 H), 0.58 (s, 3 H), 0.88 (s, 18 H), 1.12 (d, J=6.6 Hz, 3 H), 1.30-2.10 (m, 13 H), 2.22 (dd, J=7.3 and 12.9 Hz, 1 H), 2.26-2.36 (m, 1 H), 2.42-2.48 (m, 1 H), 2.81-2.87 (m, 1 H), 4.18-4.24 (m, 1 H), 4.33-4.42 (m, 1H), 4.86 (s, 1 H), 5.18 (s, 1 H), 5.79 (dd, J=8.1 and 10.0 Hz, 1 H), 5.95-6.08 (m, 2 H), 6.23 (d, J=11.2 Hz, 1 H), 6.85-7.02 (m, 2 H), 10.1 (d, J=8.1 Hz, 1 H).
MS m/z 625 (M+1)⁺.

(2) To an anhydrous THF solution (2 ml) of the above-obtained Compound (8) (A=-CH=CH-CH=CH-, PG=TBS, 24(E)) (48 mg, 76.8 µmol) were added zinc (powder) (8 mg, 0.12 mmol), methyl 2-(bromomethyl)acrylate (13.8 µl, 0.12 mmol) and a saturated ammonium chloride aqueous solution (0.7 ml) at 0°C, and the mixture was stirred for 30 min at room temperature. The reaction was quenched with water, and the mixture was extracted with ethyl acetate. The organic layer was washed with water and then with brine, dried over anhydrous magnesium sulfate, and concentrated. The residue was purified by column chromatography (5%→20% ethyl acetate in hexane) to obtain Compound (C) (60 mg, 107% yield).
Compound (C):
¹H NMR (CDCl₃) δ : 0.055 (s, 3 H), 0.059 (s, 3 H), 0.062 (s, 6 H), 0.55 (s, 3 H), 0.88 (s, 18 H), 1.04 (d, J=6.6 Hz, 3 H), 1.23-2.17 (m, 15 H), 2.18-2.24 (m, 1 H), 2.42-2.52 (m, 2 H), 2.60-2.65 (m, 1 H), 2.78-2.86 (m, 1 H), 3.74 (s, 3 H), 4.19-4.22 (m, 1 H), 4.28-4.34 (m, 1 H), 4.34-4.39 (m, 1 H), 4.86 (d, J=2.2 Hz, 1 H), 5.17 (s, 1 H), 5.53-5.62 (m, 1 H), 5.67 (d, J=1.2 Hz, 1 H), 5.90-6.03 (m, 2 H), 6.15-6.25 (m, 2 H), 6.26 (d, J=1.2 Hz, 1 H).
MS m/z 747 (M+23)⁺.

(3) To an anhydrous THF solution (2 ml) of the above obtained Compound (C) (46 mg, 63.4 µmol) was added a THF solution (63 µl, 1 N, 63 µmol) of TBAF at 0°C, and the mixture was stirred for 15 min at 0°C. The reaction was quenched with brine, and the mixture was extracted with ethyl acetate. The organic layer was washed with water and then with brine, dried over anhydrous magnesium sulfate, and concentrated. To a toluene-acetonitrile (1:1) solution (2 ml) of the residue were added LiBF₄ (24 mg, 256 µmol) and an acetonitrile solution (254 µl, 1N, 254 µmol) of sulfuric acid at 0°C, and the mixture was stirred for 15 min at 0°C. The reaction was quenched with water, and the mixture was extracted with ethyl acetate. The organic layer was washed with a saturated sodium hydrogencarbonate aqueous solution and then with brine, dried over anhydrous magnesium sulfate, and concentrated. The residue was purified by preparative TLC (hexane : ethyl acetate=1:2) and HPLC (reversed phase, elution: A; 95% H₂O/CH₃CN, B; 95% MeOH/H₂O, B=83%) to obtain the objective Compound No. 51a (the lower polarity body) (3.5 mg, 3.5% yield) in purity of 99% and Compound No. 51b (the higher polarity body) (6.3 mg, 6.3% yield) in purity of 99%. Compound No. 51a (the lower polarity body) and Compound No. 51b (the higher polarity body) are diastereoisomers based on the asymmetric point on the lactone ring of the side-chain.
Compound No. 51a (the lower polarity body):
¹H NMR (CDCl₃) δ : 0.56 (s, 3 H), 1.05 (d, J=6.3 Hz, 3 H), 1.10-2.05 (m, 15 H), 2.07-2.22 (m, 1 H), 2.31 (dd, J=6.3 and 13.4 Hz, 1 H), 2.60 (dd, J=2.9 and 13.2 Hz, 1 H), 2.70 (dtt, J=2.7, 6.4 and 17.1 Hz, 1 H), 2.83 (dd, J=3.4 and 12.2 Hz, 1 H), 3.13 (ddt, J=2.7, 7.8 and 17.1 Hz, 1 H), 4.18-4.33 (br., 1 H), 4.38-4.53 (br., 1 H), 4.93-5.03 (m, 2 H), 5.33 (s, 1 H), 5.55 (dd, J=7.1 and 15.1 Hz, 1 H), 5.62 (m, 2 H), 5.90-6.05 (m, 2 H), 6.20-6.32 (m, 2 H), 6.37 (d, J=11.2 Hz, 1 H).
MS m/z 465(M+1)⁺.
Compound No. 51b (the higher polarity body):
¹H NMR (CDCl₃) δ : 0.56 (s, 3 H), 1.05 (d, J=6.6 Hz, 3 H), 1.10-2.05 (m, 15 H), 2.10-2.20 (m, 1 H), 2.31 (dd, J=6.6 and 13.4, Hz, 1 H), 2.60 (dd, J=3.4 and 13.7 Hz, 1 H), 2.72 (dtt, J=2.9, 6.4 and 13.9 Hz, 1 H), 2.83 (dd, J=3.6 and 12.2 Hz, 1 H), 3.13 (ddt, J=2.5, 7.8 and 17.1 Hz, 1 H), 4.18-4.30 (br., 1 H), 4.38-4.50 (br., 1 H), 4.90-5.05 (m, 2 H), 5.33 (s, 1 H), 5.55 (dd, J=7.1 and 15.1 Hz, 1 H), 5.62-5.72 (m, 2 H), 5.90-6.05 (m, 2 H), 6.20-6.32 (m, 2 H), 6.37 (d, J=11.2 Hz, 1 H).
MS m/z 465(M+1)⁺.

### Example 4

### Production of 20(R)-(2-(tetrahydro-3-vinylidene-2-furanon-5-yl)-1-ethynyl)-9,10-secopregna-5(Z),7(E),10(19)-triene-1(S),3(R)-diol (Compound No. 61)

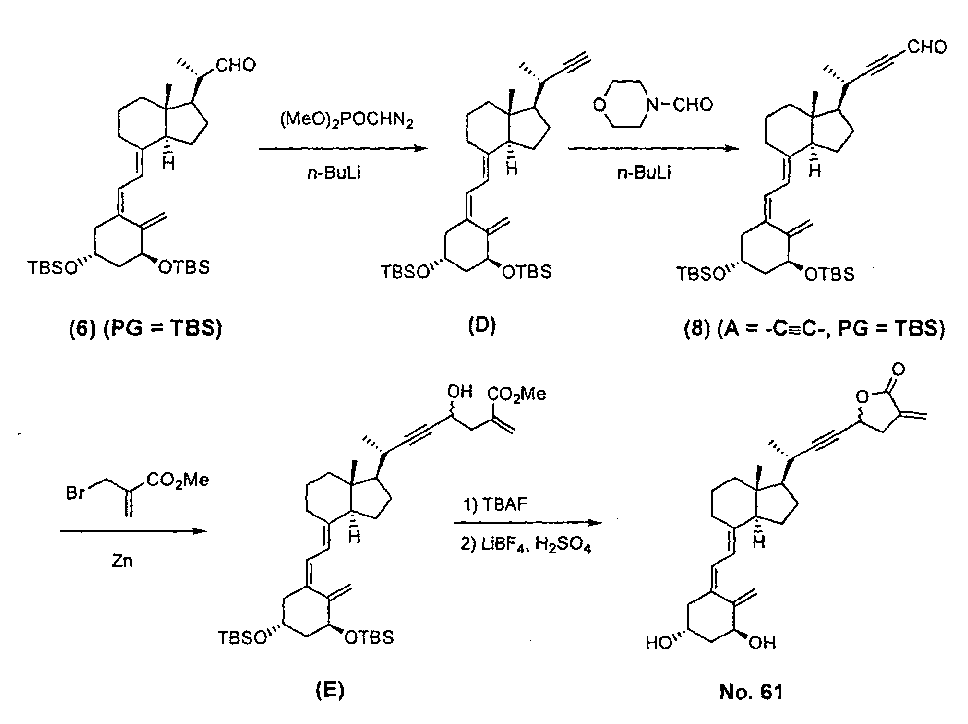

(1) To an anhydrous THF solution (0.5 ml) of n-BuLi (0.396 ml, 1.6 M in hexane, 0.634 mmol) was added dropwise dimethyl diazomethylphosphonate (89 mg, 0.594 mmol) at -78°C, and the mixture was stirred for 10 min at this temperature. To the solution was added dropwise at -78°C an anhydrous THF solution (2.5 ml) of 227 mg (0.396 mmol) of Compound (6) (PG=TBS), which had been produced by a known method (Tetrahedron, 20, 4609-4619 (1987)), and the mixture was stirred for 2 hr at this temperature and for 64 hr at -20°C. The reaction was quenched with water, and the mixture was extracted with dichloromethane. The organic layer was washed with brine, dried over anhydrous sodium sulfate. The residue was purified by preparative TLC to obtain Compound (D) (116 mg, 52% yield).
Compound (D):
¹H NMR (CDCl₃) δ : 0.06 (s, 6 H), 0.07 (s, 6 H), 0.56 (s, 3 H), 0.87 (s, 9 H), 0.88 (s, 9 H), 1.23 (d, J=6.8 Hz, 3 H), 1.16-2.08 (m, 13 H), 2.02 (d, J=2.4 Hz, 1 H), 2.21 (dd, J=7.1 and 13.2 Hz, 1 H), 2.40-2.55 (m, 2 H), 2.83 (dd, J=3.7 and 12.2 Hz, 1 H), 4.14-4.24 (m, 1 H), 4.37 (dd, J=3.9 and 6.6 Hz, 1 H), 4.86 (d, J=2.0 Hz, 1 H), 5.18 (d, J=1.2 Hz, 1 H), 6.03 (d, J=11.2 Hz, 1 H), 6.23 (d, J=11.2 Hz, 1 H).
MS m/z 569(M+1)⁺.

(2) To an anhydrous hexane solution (0.5 ml) of the above obtained Compound (D) (33 mg, 58 µmol) was added dropwise n-BuLi (40 µl, 1.6 M in hexane, 64 µmol) at -78°C, and the mixture was stirred for 10 min at -78°C. Successively, N-formylmorpholine (7 µl, 70 µmol) was added dropwise, and the mixture was stirred for 1 hr at 0°C. The reaction was quenched with a saturated ammonium chloride aqueous solution, and the mixture was extracted with diethyl ether. The organic layer was dried over anhydrous sodium sulfate and concentrated. The residue was purified by preparative TLC to obtain Compound (8) (A=-C≡C-, PG=TBS) (30 mg, 87% yield).
Compound (8):
¹H NMR (CDCl₃) δ : 0.059 (s, 3 H), 0.062 (s, 6 H), 0.07 (s, 3 H), 0.57 (s, 3 H), 0.87 (s, 9 H), 0.88 (s, 9 H), 1.29 (d, J=6.8 Hz, 3 H), 1.25-2.10 (m, 13 H), 2.22 (dd, J=6.8 and 12.7 Hz, 1 H), 2.44 (dd, J=3.7 and 12.7 Hz, 1 H), 2.65-2.75 (m, 1 H), 2.80-2.87 (m, 1 H), 4.15-4.24 (m, 1 H), 4.38 (dd, J=4.1 and 6.8 Hz, 1 H), 4.86 (d, J=2.0 Hz, 1 H), 5.19 (d, J=1.2 Hz, 1 H), 6.02 (d, J=11.2 Hz, 1 H), 6.22 (d, J=11.2 Hz, 1 H), 9.18 (d, J=0.73 Hz, 1 H).
MS m/z 597(M+1)⁺.

(3) To an anhydrous THF solution (1 ml) of the above-obtained Compound (8) (A=-C≡C-, PG=TBS) (89 mg, 0.149 mmol) were added zinc (powder) (15 mg, 0.22 mmol), methyl 2-(bromomethyl)acrylate (27 µl, 0.22 mmol) and a saturated ammonium chloride aqueous solution (1.4 ml), and the mixture was stirred for 15 min at room temperature. The reaction was quenched with water, and the reaction mixture was extracted with ethyl acetate. The organic layer was washed with water and brine, dried over anhydrous magnesium sulfate, and concentrated. The residue was purified by preparative TLC (hexane : ethyl acetate=4:1) to obtain Compound (E) (99 mg, 95% yield).
Compound (E):
¹H NMR (CDCl₃) δ : 0.058 (s, 3 H), 0.062 (s, 6 H), 0.07 (s, 3 H), 0.54 (s, 3 H), 0.87 (s, 9 H), 0.88 (s, 9 H), 1.18 (d, J=6.8 Hz, 3 H), 1.20-2.05 (m, 13 H), 2.21 (dd, J=7.3 and 13.2 Hz, 1 H), 2.29 (dd, J=2.4 and 5.9 Hz, 1 H), 2.40-2.55 (m, 2 H), 2.69 (d, J=6.3 Hz, 2 H), 2.79-2.87 (m, 1 H), 3.77 (s, 3 H), 4.13-4.23 (m, 1 H), 4.37 (dd, J=4.4 and 7.1 Hz, 1 H), 4.52-4.60 (m, 1 H), 4.86 (d, J=2.0 Hz, 1 H), 5.19 (d, J=1.7 Hz, 1 H), 5.73 (d, J=1.2 Hz, 1 H), 6.02 (d, J=11.2 Hz, 1 H), 6.22 (d, J=11.2 Hz, 1 H), 6.29 (d, J=1.2 Hz, 1 H).
MS m/z 697(M+1)⁺.

(4) To an anhydrous THF solution (1.5 ml) of the above-obtained Compound (E) (89 mg, 0.128 mmol) was added a THF solution (128 µl, 1 N, 0.128 mmol) of TBAF at 0°C, and the mixture was stirred for 100 min at 0°C. The reaction was quenched with brine, and the mixture was extracted with ethyl acetate. The organic layer was washed with water and brine, dried over anhydrous magnesium sulfate, and concentrated. To a toluene-acetonitrile (1:1) solution (2 ml) of the residue were added LiBF₄ (48 mg, 0.512 mmol) and an acetonitrile solution (512 µl, 1N, 0.512 mmol) of sulfuric acid at 0°C, and the mixture was stirred for 15 min at 0°C. The reaction was quenched with water, and the mixture was extracted with ethyl acetate. The organic layer was washed with a saturated sodium hydrogencarbonate aqueous solution and brine, dried over magnesium sulfate, and concentrated. The residue was purified by preparative TLC (hexane : ethyl acetate=1:3) and HPLC (reversed phase, elution: A; 95% H₂O/CH₃CN, B; 95% CH₃CN-MeOH (5:3)/H₂O, B=75%) to obtain the objective Compound No. 61 (6.2 mg, 25% yield) in purity of 95%.
Compound No. 61:
¹H NMR (CDCl₃) δ : 0.52 (s, 3 H), 1.20 (d, J=7.1 Hz, 3 H), 1.00-2.05 (m, 15 H), 2.31 (dd, J=6.3 and 13.4 Hz, 1 H), 2.45-2.58 (m, 1 H), 2.60 (dd, J=3.7 and 13.4 Hz, 1 H), 2.83 (dd, J=4.1 and 12.2 Hz, 1 H), 1.92 (ddt, J=2.9, 5.9 and 16.8 Hz, 1 H), 3.21 (ddt, J=2.4, 8.3 and 16.8 Hz, 1 H), 4.18-4.28 (m, 1 H), 4.40-4.48 (m, 1 H), 4.99-5.00 (m, 1 H), 5.08-5.18 (m, 1 H), 5.33-5.34 (m, 1 H), 5.63-5.70 (m, 1 H), 6.02 (d, J=11.2 Hz, 1 H), 6.24-6.29 (m, 1 H), 6.37 (d, J=11.2 Hz, 1 H).
MS m/z 437(M+1)⁺.

### Example 5

### Production of 20(R)-(2-(tetrahydro-3-vinylidene-2-furanon-5-yl)-1-propynyl)-9,10-secopregna-5(Z),7(E),10(19)-triene-1(S),3(R)-diol (Compound No. 71)

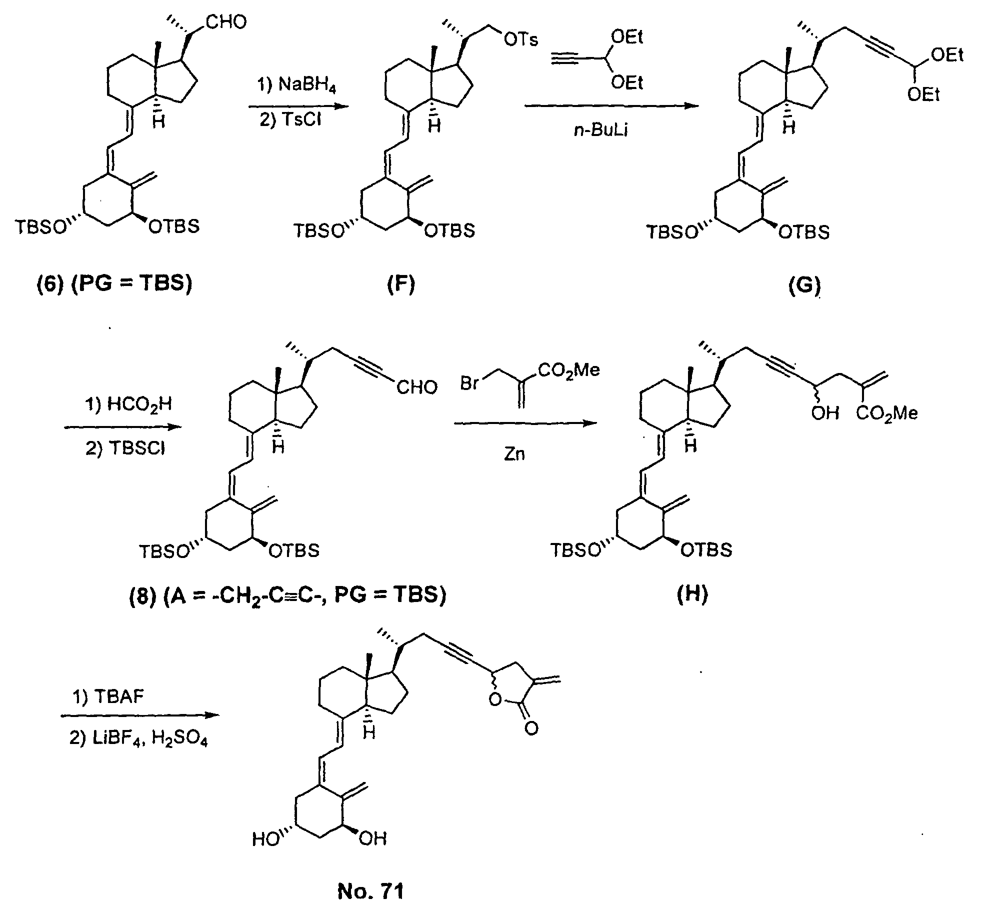

(1) To a 1,4-dioxane solution (3 ml) of propargyl aldehyde diethyl acetal (81 µl, 0.56 mmol) was added dropwise n-BuLi (0.35 ml, 1.6 M in hexane, 0.56 mmol) at 5°C, and the mixture was stirred for 10 min at 5°C and for 20 min at room temperature. To this solution was added a dioxane solution (3 ml) of Compound (F) (102 mg, 0.14 mmol) obtained from Compound (6) (PG=TBS) in the same way as described in Example 10, and the mixture was heated and refluxed for 14 hr. The reaction was quenched with a saturated sodium hydrogencarbonate solution, and the mixture was extracted with ethyl acetate. The organic layer was washed with water and brine, dried over anhydrous magnesium sulfate, and concentrated. The residue was purified by preparative TLC (hexane : ethyl acetate=9:1) to obtain Compound (G) (60 mg, 63% yield).
Compound (G):
¹H NMR (CDCl₃) δ : 0.057 (s, 6 H), 0.062 (s, 6 H), 0.53 (s, 3 H), 0.88 (s, 18 H), 1.09 (d, J=6.6 Hz, 3 H), 1.12-2.12 (m, 21 H), 2.21 (dd, J=7.3 and 14.1 Hz, 1 H), 2.28-2.38 (m, 1 H), 2.44 (dd, J=3.7 and 13.2 Hz, 1 H), 2.82 (d, J=3.7 and 12.9 Hz, 1 H), 3.53-3.63 (m, 2 H), 3.70-3.80 (m, 2 H), 4.15-4.23 (m, 1 H), 4.34-4.38 (dd, J=3.6 and 6.6 Hz, 1 H), 4.86 (d, J=2.2 Hz, 1 H), 5.18 (d, J=1.5 Hz, 1 H), 5.27 (s, 1 H), 6.02 (d, J=11.2 Hz, 1 H), 6.23 (d, J=11.2 Hz, 1 H).
MS m/z 685(M+1)⁺.

(2) To a THF-H₂O (1:1) solution (2 ml) of the above-obtained Compound (G) (18 mg, 26.3 µmol) was added formic acid, and the mixture was stirred for 3 days at 40°C. The reaction was quenched with a saturated sodium hydrogencarbonate aqueous solution, and the mixture was extracted with ethyl acetate. The organic layer was washed with brine, dried over anhydrous magnesium sulfate and concentrated. To a DMF solution of the residue were added imidazole (11 mg, 0.16 mmol) and TBSCl (112 mg, 79 µmol), and the mixture was stirred for 3 hr at room temperature. The reaction mixture was extracted with ethyl acetate, and the organic layer was washed with brine, dried over anhydrous magnesium sulfate and concentrated. The residue was purified by preparative TLC (hexane : ethyl acetate=10:1) to obtain Compound (8) (A=-CH2-C≡C-, PG=TBS) (4.7 mg, 29% yield).
Compound (8) (A=-CH2-C≡C-, PG=TBS):
¹H NMR (CDCl₃) δ : 0.059 (s, 6 H), 0.065 (s, 6 H), 0.55 (s, 3 H), 0.88 (s, 18 H), 1.12 (d, J=6.6 Hz, 3 H), 1.20-2.05 (m, 14 H), 2.22 (dd, J=7.3 and 12.9 Hz, 1 H), 2.29 (dd, J=7.6 and 12.4 Hz, 1 H), 2.43-2.53 (m, 2 H), 2.83 (dd, J=4.4 and 12.4 Hz, 1 H), 4.16-4.23 (m, 1 H), 4.37 (dd, J=3.7 and 6.8 Hz, 1 H), 4.86 (d, J=2.2 Hz, 1 H), 5.18 (d, J=1.5 Hz, 1 H), 6.02 (d, J=11.2 Hz, 1 H), 6.23 (d, J=11.2 Hz, 1 H), 9.20 (s, 1 H).
MS m/z 611(M+1)⁺.

(3) To an anhydrous THF solution of the above-obtained Compound (8) (A=-CH2-C≡C-, PG=TBS) (40 mg, 65.5 µmol) were added zinc (powder) (6.4 mg, 98 µmol), methyl 2-(bromomethyl)acrylate (12 µl, 98 µmol) and a saturated ammonium chloride aqueous solution (0.6 ml) at 0°C, and the mixture was stirred for 30 min at 0°C. The reaction was quenched with water, and the mixture was extracted with ethyl acetate. The organic layer was washed with water and brine, dried over anhydrous magnesium sulfate and concentrated. The residue was purified by preparative TLC (hexane : ethyl acetate=3:1) to obtain Compound (H) (25 mg, 54% yield).
Compound H:
¹H NMR (CDCl₃) δ : 0.057 (s, 6 H), 0.063 (s, 6 H), 0.53 (s, 3 H), 0.88 (s, 18 H), 1.05 (d, J=6.3 Hz, 3 H), 1.20-2.50 (m, 19 H), 2.70-2.75 (m, 1 H), 2.78-2.87 (m, 1 H), 3.78 (s, 3H), 4.15-4.22 (m, 1 H), 4.34-4.40 (m, 1 H), 4.55-4.62 (m, 1 H), 4.86 (d, J=2.2 Hz, 1 H), 5.16-5.19 (m, 1 H), 5.75 (d, J=1.2 Hz, 1 H), 6.02 (d, J=11.7 Hz, 1 H), 6.23 (s, J=11.7 Hz, 1 H), 6.30 (d, J=1.2 Hz, 1 H).
MS m/z 711(M+1)⁺

(4) To an anhydrous THF solution of the above-obtained Compound (H) (30 mg, 43 µmol) was added a THF solution (43 µl, 1 N, 43 µmol) of TBAF at 0°C, and the mixture was stirred for 30 min at 0°C. The reaction was quenched with brine, and the mixture was extracted with ethyl acetate, The organic layer was washed with water and brine, dried over anhydrous magnesium sulfate, and concentrated. To a toluene-acetonitrile (1:1) solution (1 ml) of the residue were added LiBF₄ (16 mg, 0.170 mmol) and an acetonitrile solution (170 µl, 1 N, 0.170 mmol) of sulfuric acid at 0°C, and the mixture was stirred for 10 min at 0°C. The reaction was quenched with water, and the mixture was extracted with ethyl acetate. The organic layer was washed with a saturated sodium hydrogencarbonate aqueous solution and brine, dried over magnesium sulfate, and concentrated. The residue was purified by HPLC (reversed phase, elution: A; 95% H₂O/CH₃CN, B; 95% CH₃CN/H₂O, B=60%) to obtain the objective Compound No. 71 (7.3 mg, 38% yield) in purity of 94%.
Compound No. 71:
¹H NMR (CDCl₃) δ : 0.55 (s, 3 H), 1.06 (d, J=6.3 Hz, 3 H), 1.09-2.10 (m, 16 H), 2.28-2.54 (m, 2 H), 2.60 (dd, J=2.9 and 13.4 Hz, 1 H), 2.82 (dd, J=4.1 and 12.7 Hz, 1 H), 2.96 (dtt, J=2.7, 5.6 and 16.8 Hz, 1 H), 3.24 (ddt, J=2.4, 8.3 and 16.8 Hz, 1 H), 3.68-3.77 (m, 1 H), 4.18-4.28 (m, 1 H), 4.40-4.48 (m, 1 H), 5.00 (s, 1 H), 5.12-5.20 (m, 1 H), 5.33 (s, 1 H), 5.65-5.70 (m, 1 H), 6.02 (d, J=11.2 Hz, 1 H), 6.25-6.30 (m, 1 H), 6.38 (d, J=11.2 Hz, 1 H).
MS m/z 451(M+1)⁺.

### Example 6

### Production of 2 (S)-methyl-20(R)-(tetrahydro-3-vinylidene-2-furanon-5-(S)-yl)methyl-9,10-secopregna-5(Z),7(E),10(19)-triene-1(R),3(S)-diol (Compound No. 22a)

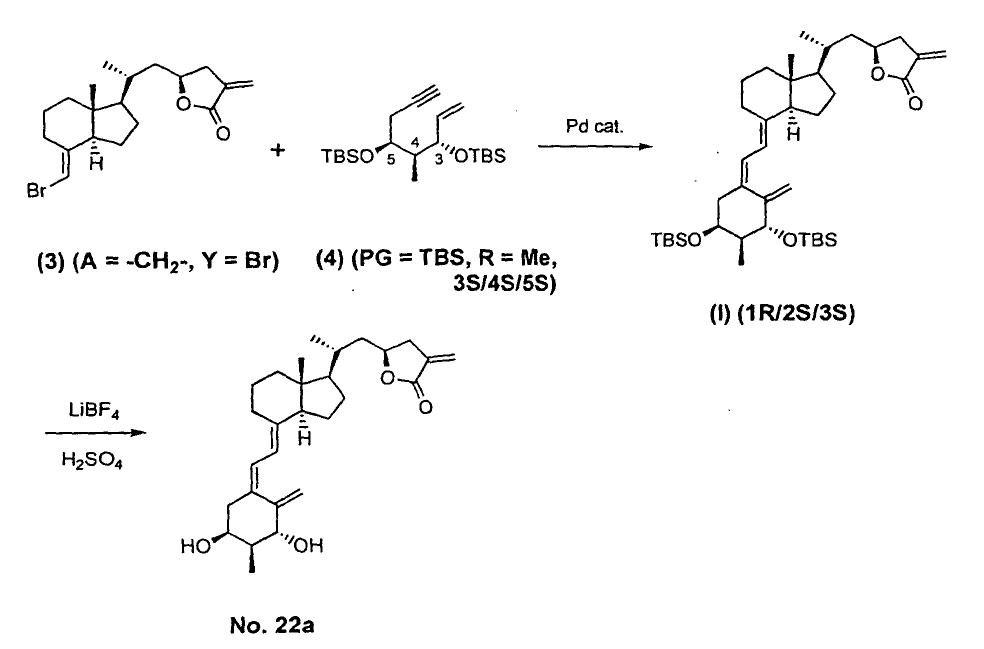

Pd₂(dba)₃ ·CHCl₃ (8.1 mg, 7.8 µmol) and triphenylphosphine (21 mg, 78 µmol) were dissolved in anhydrous toluene (0.5 ml) under a nitrogen atmosphere, and the solution was stirred for 15 min at room temperature. To the solution was added an anhydrous toluene solution (0.5 ml) of Compound (3) (A=-CH₂-, Y=Br) (29 mg, 78 µmol), which had been manufactured by a method described in the specification of WO95/33716, and Compound (4) (PG=TBS, R=Me, 3S/4S/5S), an ene-yne compound, (29 mg, 7.8 µmol), which had been manufactured by a method described in J. Med. Chem., 43, 4247-4265 (2000), and successively anhydrous triethylamine (1.0 ml) was added. The mixture was heated and stirred for 8 hr at 100°C. The reaction mixture was extracted with ethyl acetate after the addition of a saturated potassium sulfite aqueous solution. The organic layer was washed with brine, dried and concentrated. The residue was purified by silica gel column chromatography (hexane : ethyl acetate=40:1 to 20:1) to obtain Compound (I) (1R/2S/3S) (19 mg, a pale yellow oil).
Compound (I) (1R/2S/3S):
MS m/z 669.5 (M+1)⁺.

The obtained Compound (I) (1R/2S/3S) (19 mg, 28 µmol) was dissolved in a mixed solvent of acetonitrile (1 ml) and methylene chloride (1 ml), LiBF₄ (8 mg, 85 µmol) was added to the solution, and the solution was ice-cooled. To the solution was added an acetonitrile solution (51 µl, 1 N, 51 µmol) of sulfuric acid, and the mixture was stirred for 40 min under ice cooling. Subsequently, an acetonitrile solution (51 µl, 1 N, 51 µmol) of sulfuric acid was further added every 30 min three times in total, and the mixture was stirred for 2.5 hr in total under ice cooling. The reaction mixture was extracted with ethyl acetate after the addition of a saturated sodium hydrogencarbonate aqueous solution. The organic layer was washed with brine, dried and concentrated. The residue was purified by a silica gel column cartridge (manufactured by Waters Co., Sep-pak Plus Silica Cartridge, Hexane : ethyl acetate=3:1→hexane : ethyl acetate=1:1→ hexane : ethyl acetate : methanol=3:3:1) and HPLC (reversed phase, elution: A; 95% H₂O/CH₃CN, B; 95% MeOH/H₂O, B=80%) to obtain the objective Compound No. 22a (1.9 mg, 5.5% yield) in purity of 99.2%.
Compound No. 22a:
¹H NMR (CDCl₃) δ : 0.55 (s, 3 H), 1.03 (d, J=6.3 Hz, 3 H), 1.13 (d, J=6.8 Hz, 3 H), 1.1-1.8 (m, 13 H), 1.84-2.02 (m, 4 H), 2.42 (dd, J=5.9 and 13.9 Hz, 1 H), 2.51-2.57 (m, 2 H), 2.82-2.85 (m, 1 H), 3.02-3.08 (m, 1 H), 4.06 (br., 2 H), 4.59 (quint, J=6.8 Hz, 1 H), 5.01 (s, 1 H), 5.35 (s, 1 H), 5.62 (t, J=2.4 Hz, 1 H), 6.01 (d, J=11.2 Hz, 1 H), 6.23 (t, J=2.7 Hz, 1 H), 6.35 (d, J=11.2 Hz, 1 H).
MS m/z 441.2 (M+1)⁺.

### Example 7

### Production of 2(S)-methyl-20(R)-(tetrahydro-3-vinylidene-2-furanon-5-(S)-yl)methyl-9,10-s ecopregna-5(Z),7(E),10(19)-triene-1(S),3(S)-diol (Compound No. 22b)

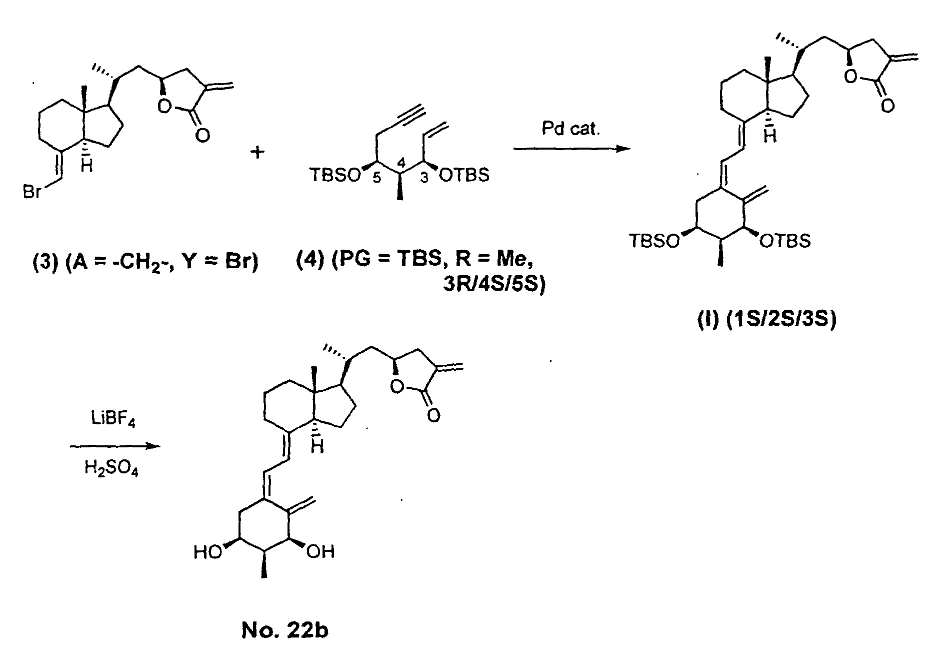

Pd₂(dba)₃·CHCl₃ (8.1 mg, 7.8 µmol) and triphenylphosphine (21 mg, 78 µmol) were dissolved in anhydrous toluene (0.5 ml) under a nitrogen atmosphere, and the solution was stirred for 15 min at room temperature. To the solution was added an anhydrous toluene solution (0.5 ml) of Compound (3) (A=-CH₂-, Y=Br) (29 mg, 78 µmol), which had been manufactured by a method described in the specification of WO95/33716, and Compound (4) (PG=TBS, R=Me, 3R/4S/5S), an ene-yne compound, (29 mg, 7.8 µmol), which had been manufactured by a method described in J. Med. Chem., 43, 4247-4265 (2000), and successively anhydrous triethylamine (1.0 ml) was added. The solution was heated and stirred for 6 hr at 100°C. The reaction mixture was extracted with ethyl acetate after the addition of a saturated potassium sulfite aqueous solution. The organic layer was washed with brine, dried and concentrated. The residue was purified by silica gel column chromatography (hexane : ethyl acetate=40:1 to 20:1) to obtain Compound (I) (1S/2S/3S) (36 mg, a pale yellow oil).
Compound (I) (1S/2S/3S):
MS m/z 669.5 (M+1)⁺.

The obtained Compound (I) (1S/2S/3S) (36 mg, 54 µmol) was dissolved in a mixed solvent of acetonitrile (1 ml) and methylene chloride (1 ml), LiBF₄ (15 mg, 161 µmol) was added to the solution, and the solution was ice-cooled. To the solution was added an acetonitrile solution (97 µl, 1 N, 97 µmol) of sulfuric acid, and the mixture was stirred for 40 min under ice cooling. The reaction mixture was extracted with ethyl acetate after the addition of a saturated sodium hydrogencarbonate aqueous solution. The organic layer was washed with brine, dried and concentrated. The residue was purified by a silica gel column cartridge (manufactured by Waters Co., Sep-pak Plus Silica Cartridge, Hexane : ethyl acetate=3:1→hexane : ethyl acetate=1:1→hexane : ethyl acetate : methanol=3:3:1) and HPLC (reversed phase, elution: A; 95% H₂O/CH₃CN, B; 95% MeOH/H₂O, B=80%) to obtain the objective Compound No. 22b (3.2 mg, 9.3% yield, colorless film) in purity of 100%.
Compound No. 22b:
¹H NMR (CDCl₃) δ : 0.55 (s, 3 H), 1.03 (d, J=6.3 Hz, 3 H), 1.22 (d, J=7.3 Hz, 3 H), 1.1-1.8 (m, 11 H), 1.92-2.05 (m, 4 H), 2.17 (br., 1 H), 2.49-2.60 (m, 3 H), 2.79-2.88 (m, 2 H), 3.02-3.09 (m, 1 H), 3.92 (br., 1 H), 4.17 (br., 1 H), 4.59 (quint, J=6.8 Hz, 1 H), 4.98 (s, 1 H), 5.23 (s, 1 H), 5.62 (s, 1 H), 6.03 (d, J=11.2 Hz, 1 H), 6.23 (s, 1 H), 6.47 (d, J=11.5 Hz, 1 H).
MS m/z 441.2 (M+1)⁺.

### Example 8

### Production of 2(S)-methyl-20(R)-(tetrahydro-3-vinylidene-2-furanon-5-(S)-yl)methyl-9,10-s ecopregna-5(Z),7(E),10(19)-triene-1(R),3(R)-diol (Compound No. 22c)

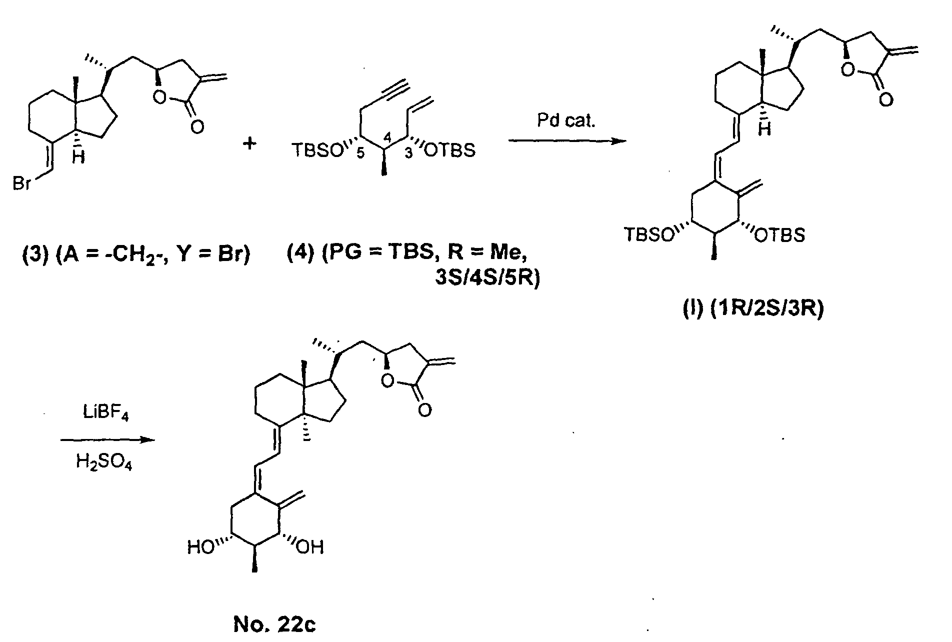

Pd₂(dba)₃·CHCl₃ (8.1 mg, 7.8 µmol) and triphenylphosphine (21 mg, 78 µmol) were dissolved in anhydrous toluene (0.5 ml) under a nitrogen atmosphere, and the solution was stirred for 15 min at room temperature. To the solution was added an anhydrous toluene solution (0.5 ml) of Compound (3) (A=-CH₂-, Y=Br) (29 mg, 78 µmol), which had been manufactured by a method described in the specification of WO95/33716, and Compound (4) (PG=TBS, R=Me, 3S/4S/5R) (29 mg, 7.8 µmol), an ene-yne compound, which had been manufactured by a method described in J. Med. Chem., 43, 4247-4265 (2000), and successively anhydrous triethylamine (1.0 ml) was added. The solution was heated and stirred for 9 hr at 100°C. The reaction mixture was extracted with ethyl acetate after the addition of a saturated potassium sulfite aqueous solution. The organic layer was washed with brine, dried and concentrated. The residue was purified by silica gel column chromatography (hexane : ethyl acetate=40:1 to 20:1) to obtain Compound (I) (1R/2S/3R) (20 mg, a pale yellow oil).
Compound (I) (1R/2S/3R):
MS m/z 669.5 (M+1)⁺.

The obtained Compound (I) (1R/2S/3R) (20 mg, 30 µmol) was dissolved in a mixed solvent of acetonitrile (1 ml) and methylene chloride (1 ml), LiBF₄ (8.4 mg, 90 µmol) was added to the solution, and the solution was ice-cooled. To the solution was added an acetonitrile solution (54 µl, 1 N, 54 µmol) of sulfuric acid, and the mixture was stirred for 50 min under ice cooling. The reaction mixture was extracted with ethyl acetate after the addition of a saturated sodium hydrogencarbonate aqueous solution. The organic layer was washed with brine, dried and concentrated. The residue was purified by a silica gel column cartridge (manufactured by Waters Co., Sep-pak Plus Silica Cartridge, Hexane : ethyl acetate=3:1→hexane : ethyl acetate=1:1→hexane : ethyl acetate : methanol=3:3:1) and HPLC (reversed phase, elution: A; 95% H₂O/CH₃CN, B; 95% MeOH/H₂O, B=78%) to obtain the objective Compound No. 22c (1.0 mg, 2.9% yield) in purity of 99%.
Compound No. 22c:
¹H NMR (CDCl₃) δ : 0.57 (s, 3 H), 1.03 (d, J=7.1 Hz, 3 H), 1.04 (d, J=6.3 Hz, 3 H), 1.1-1.7 (m, 11 H), 1.88-2.12 (m, 4 H), 2.29 (br., 1 H), 2.36 (dd, J=5.4 and 13.9 Hz, 1 H), 2.51-2.57 (m, 1 H), 2.64-2.68 (m, 1 H), 2.75 (br., 1 H), 2.83-2.86 (m, 1 H), 3.02-3.08 (m, 1 H), 3.72 (br., 1 H), 3.96 (br., 1 H), 4.59 (quint, J=7.1 Hz, 1 H), 5.06 (s, 1 H), 5.30 (s, 1 H), 5.62 (s, 1 H), 6.05 (d, J=11.5 Hz, 1 H), 6.23 (s, 1 H), 6.42 (d, J=11.2 Hz, 1 H).
MS m/z 441.2 (M+1)⁺.

### Example 9

### Production of 2(S)-methyl-20(R)-(tetrahydro-3-vinylidene-2-furanon-5-(S)-yl)methyl-9,10-s ecopregna-5(Z),7(E),10(19)-triene-1(S),3(R)-diol (Compound No. 22d)

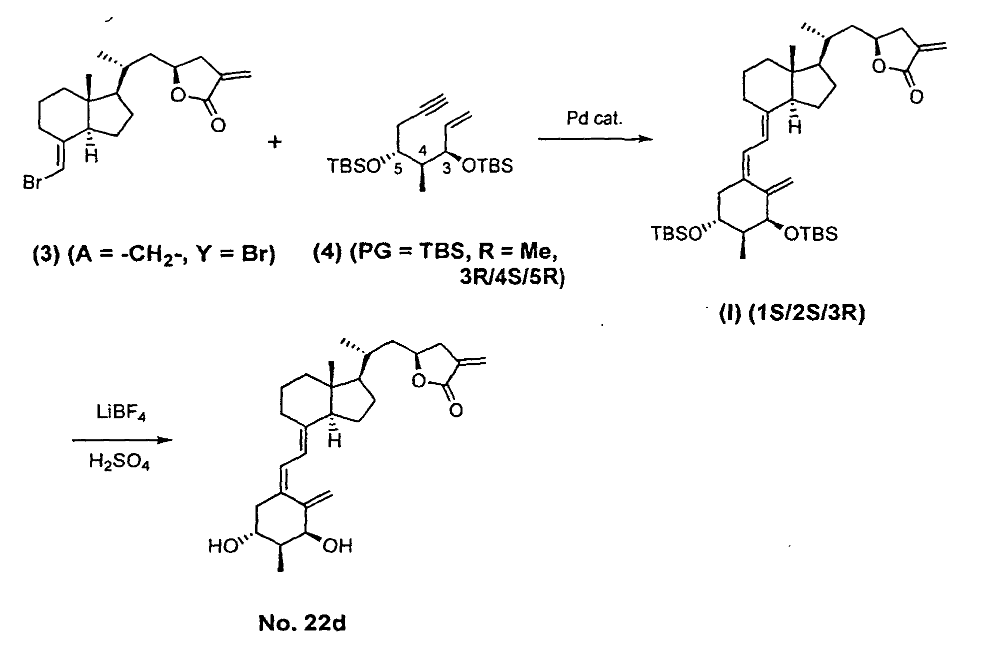

Pd₂(dba)₃ ·CHCl₃ (8.1 mg, 7.8 µmol) and triphenylphosphine (21 mg, 78 µmol) were dissolved in anhydrous toluene (0.5 ml) under a nitrogen atmosphere, and the solution was stirred for 15 min at room temperature. To the solution was added an anhydrous toluene solution (0.5 ml) of Compound (3) (A=-CH₂-, Y=Br) (29 mg, 78 µmol), which had been manufactured by a method described in the specification of WO95/33716, and Compound (4) (PG=TBS, R=Me, 3R/4S/5R) (29 mg, 7.8 µmol), an ene-yne compound, which had been manufactured by a method described in J. Med. Chem., 43, 4247-4265 (2000), and successively anhydrous triethylamine (1.0 ml) was added. The solution was heated and stirred for 9 hr at 100°C. The reaction mixture was extracted with ethyl acetate after the addition of a saturated potassium sulfite aqueous solution. The organic layer was washed with brine, dried and concentrated. The residue was purified by silica gel column chromatography (hexane : ethyl acetate=40:1 to 20:1) to obtain Compound (I) (1S/2S/3R) (23 mg, a pale yellow oil).
Compound (I) (1S/2S/3R):
MS m/z 669.5 (M+1)⁺.

The obtained Compound (I) (1S/2S/3R) (23 mg, 34 µmol) was dissolved in a mixed solvent of acetonitrile (1 ml) and methylene chloride (1 ml), LiBF₄ (10 mg, 103 µmol) was added to the solution, and the mixture was ice cooled. To the solution was added an acetonitrile solution (62 µl, 1 N, 62 µmol) of sulfuric acid, and the mixture was stirred for 50 min under ice cooling. Since the reaction had not been completed at this point, an acetonitrile solution (62 µl, 1 N, 62 µmol) of sulfuric acid was further added, and the mixture was stirred for 40 min under ice cooling and for 20 min at room temperature. Since the reaction had not been completed again at this point, an acetonitrile solution (62 µl, 1 N, 62 µmol) of sulfuric acid was further added, and the mixture was stirred for 40 min at room temperature. The reaction mixture was extracted with ethyl acetate after the addition of a saturated sodium hydrogencarbonate aqueous solution. The organic layer was washed with brine, dried and concentrated. The residue was purified by a silica gel column cartridge (manufactured by Waters Co., Sep-pak Plus Silica Cartridge, Hexane : ethyl acetate=3:1→hexane : ethyl acetate=1:1→ hexane : ethyl acetate : methanol=3:3:1) and HPLC (reversed phase, elution: A; 95% H₂O/CH₃CN, B; 95% MeOH/H₂O, B=78%) to obtain the objective Compound No. 22d (1.4 mg, 4.1% yield, colorless film) in purity of 99%.
Compound No. 22d:
¹H NMR (CDCl₃) δ : 0.55 (s, 3 H), 1.03 (d, J=6.3 Hz, 3 H), 1.08 (d, J=6.8 Hz, 3 H), 1.1-1.8 (m, 13 H), 1.90-2.04 (m, 4H), 2.24 (dd, J=7.8 and 13.4 Hz, 1 H), 2.51-2.57 (m, 1 H), 2.67 (dd, J=4.1 and 13.4 Hz, 1H), 2.82-2.85 (m, 1 H), 3.02-3.08 (m, 1 H), 3.85 (br., 1 H), 4.31 (s, 1 H), 4.59 (quint, J=6.8 Hz, 1 H), 5.01 (s, 1 H), 5.28 (s, 1 H), 5.62 (s, 1 H), 6.01 (d, J=11.2 Hz, 1 H), 6.22 (s, 1 H), 6.39 (d, J=11.0 Hz, 1 H).
MS m/z 441.2 (M+1)⁺.

### Experiment 10

### Production of 20(R)-(2-(tetrahydro-3-vinylidene-2-furanon-5-yl)-1(E)-propenyl)-9,10-secopregna-5(Z),7(E),10(19)-triene-1(S),3(R)-diol (Compound No. 41)

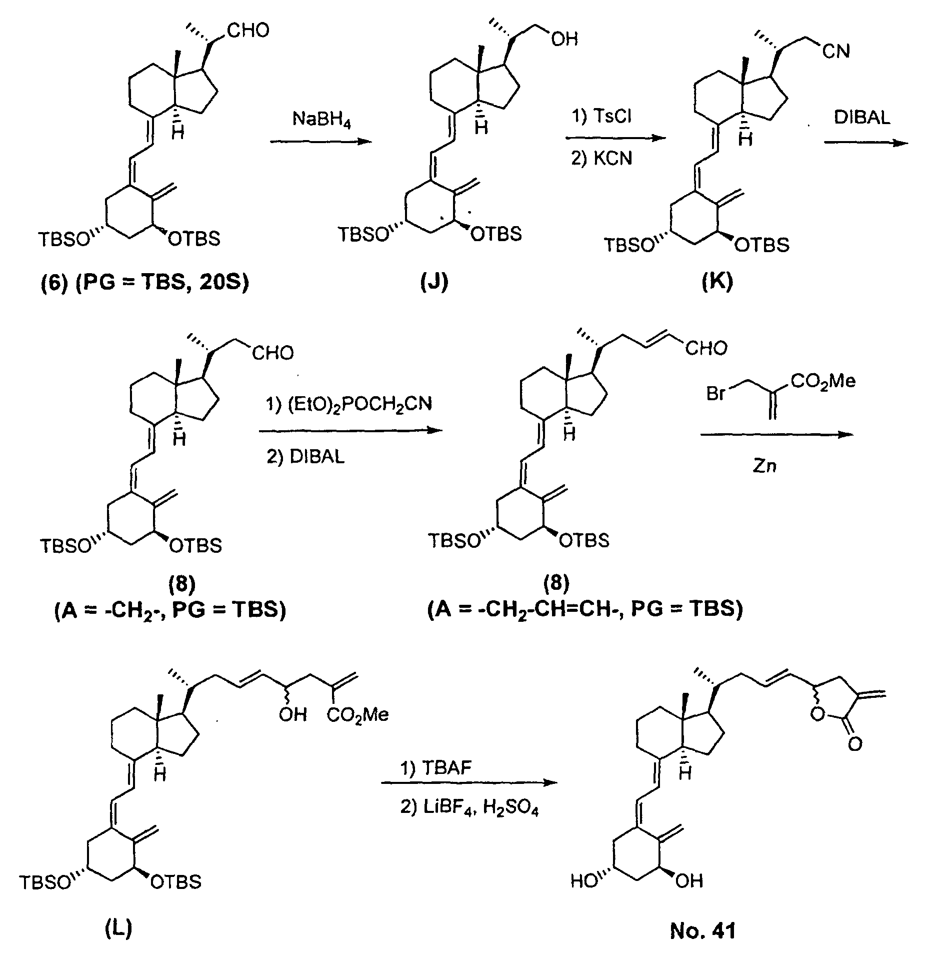

(1) Compound (6) (PG=TBS, 20S) (1.15 g, 2.0 mmol) obtained by a known method (Tetrahedron, 20, 4609-4619, (1987)) was dissolved in a mixed solvent of THF (10 ml) and MeOH (10 ml), and the solution was ice-cooled. To the solution was added sodium borohydride (38 mg, 2.0 mmol), and the mixture was stirred for 1.5 hr under ice cooling. The reaction mixture was concentrated to about half the volume after the addition of a saturated ammonium chloride aqueous solution. The concentrate was extracted with ethyl acetate, and the organic layer was washed with brine, dried and concentrated. The residue was purified by silica gel column chromatography (hexane : ethyl acetate=20:1 to 15:1) to obtain Compound (J) (200 mg, 17% yield).
(2) The above-obtained Compound (J) (200 mg, 0.348 mmol) was dissolved in pyridine (1.5 ml), tosyl chloride (133 mg, 0.696 mmol) was added to the solution, and the mixture was stirred for 7.5 hr at room temperature. The reaction mixture was extracted with ethyl acetate after the addition of 1 N hydrochloric acid. The organic layer was washed with brine, dried and concentrated to obtain a crude tosyl body (257 mg). This tosyl body was dissolved in anhydrous DMF (3 ml), potassium cyanide (45 mg, 0.696 mmol) and 18-crown-6 (9 mg, 0.035 mmol) were added to the solution, and the mixture was stirred for 3.5 hr at 100°C. The reaction mixture was extracted with ethyl acetate after the addition of water, and the organic layer was washed with brine, dried and concentrated. The residue was purified by silica gel column chromatography (hexane : ethyl acetate=40:1) to obtain Compound (K) (121 mg, 60% yield).
(3) The above-obtained Compound (K) (121 mg, 0.207 mmol) was dissolved in anhydrous methylene chloride (3 ml), and the solution was cooled to -75°C. To the solution was added a toluene solution of DIBAL (0.41 ml, 1.01 M, 0.414 mmol), and the mixture was stirred for 3 hr at this temperature. Further, a toluene solution of DIBAL (0.20 ml, 1.01 M, 0.402 mmol) was added, and the mixture was stirred for 3 hr while the temperature was gradually raised (-75°C→-10°C). The reaction mixture was extracted with methylene chloride after the addition of water and 6N hydrochloric acid, and the organic layer was washed with a saturated sodium hydrogencarbonate aqueous solution and brine, dried, and concentrated. The residue was purified by silica gel column chromatography (hexane : ethyl acetate=40:1) to obtain Compound (8) (A=-CH₂-, PG=TBS) (70 mg, 58% yield).
(4) Sodium hydride (35 mg, 60%, 0.86 mmol) was suspended in anhydrous THF (3 ml) under a nitrogen atmosphere, and the solution was ice-cooled. To the suspension was added diethyl cyanomethylphosphonate (173 mg, 0.98 mmol), and the mixture was stirred for 2 hr under ice cooling. To the reaction mixture was added an anhydrous THF solution (5 ml) of the above-obtained Compound (8) (A=-CH₂-, PG=TBS) (338 mg, 0.58 mmol), and the mixture was stirred for 3 hr under ice cooling. The reaction mixture was extracted with ethyl acetate after the addition of a saturated ammonium chloride aqueous solution. The organic layer was washed with brine, dried and concentrated to obtain a crude Wittig adduct (MS m/z, 610.5 (M+1)⁺). The adduct was dissolved in anhydrous methylene chloride (3 ml), and the solution was cooled to -70°C. To the solution was added a toluene solution of DIBAL (1.14 ml, 1.01 M, 1.15 mmol), and the mixture was stirred for 4 hr. During the stirring, the bath temperature was raised to -40°C. Further, a toluene solution of DIBAL (1.14 ml, 1.01 M, 1.15 mmol) was added, and the mixture was stirred for 4 hr. During the stirring, the bath temperature was raised to 10°C. The reaction mixture was extracted with methylene chloride after the addition of 1N hydrochloric acid. The organic layer was washed with a saturated sodium hydrogencarbonate aqueous solution, dried and concentrated, and the residue was purified by silica gel column chromatography (hexane : ethyl acetate=40:1 to 20:1) to obtain Compound (8) (A=-CH₂-CH=CH-, PG=TBS) (75 mg, 21% yield, colorless oil).
   Compound (8) (A=-CH₂-CH=CH-, PG=TBS):
   ¹H NMR (CDCl₃) δ : 0.06 (s, 12 H), 0.56 (s, 3H), 0.88 (s, 18 H), 0.98 (d, J=6.4 Hz, 3 H), 1.25-2.05 (m, 11 H), 2.17-2.25 (m, 3 H), 2.42-2.48 (m, 2 H), 2.7-2.8 (m, 1 H), 4.19 (br., 1 H), 4.37 (br., 1 H), 4.87 (d, J=2.5 Hz, 1 H), 5.18 (s, 1 H), 6.02 (d, J=11.2 Hz, 1 H), 6.11-6.17 (m, 1 H), 6.24 (d, J=10.9 Hz, 1 H), 6.8-6.9 (m, 1 H), 9.52 (d, J=7.9 Hz, 1 H).
   MS m/z 613.3 (M+1)⁺.
(5) The above-obtained Compound (8) (A=-CH₂-CH=CH-, PG=TBS) (75 mg, 0.122 mmol) was dissolved in anhydrous THF (3 ml), and the solution was ice-cooled. To the solution were added zinc powder (12 mg, 0.184 mmol) and methyl 2-bromomethylacrylate (33 mg, 0.184 mmol), and finally a saturated ammonium chloride aqueous solution (0.2 ml); and the mixture was stirred for 15 min under ice cooling, and for 2 hr at room temperature. The reaction mixture was extracted with ethyl acetate after the addition of water. The organic layer was washed with brine, dried and concentrated, and the residue was purified by silica gel column chromatography (hexane : ethyl acetate=20:1 to 6:1) to obtain Compound (L) (64 mg, 73% yield).
   Compound (L):
   ¹H NMR (CDCl₃) δ : 0.06 (s, 12 H), 0.53 (s, 3 H), 0.88 (s, 18 H), 1.23-2.25 (m, 20 H), 2.43-2.62 (m, 3 H), 2.82 (d, J=10.4 Hz, 1 H), 3.77 (s, 3 H), 4.08-4.37 (m, 3 H), 4.87 (d, J=2.3 Hz, 1 H), 5.18 (s, 1 H), 5.44-5.49 (m, 1 H), 5.61-5.67 (m, 2 H), 6.02 (d, J=11.2 Hz, 1 H), 6.22-6.25 (m, 2 H).
   MS m/z 713.5 (M+1)⁺, 695.5 (M-H₂O+1)⁺.
(6) The above-obtained Compound (L) (64 mg, 90 µmol) was dissolved in anhydrous THF (3 ml), and the solution was ice-cooled. To the solution was added a THF solution (270 µl, 1 N, 270 µmol) of tetrabutylammonium fluoride, and the mixture was stirred for 1 hr under ice cooling. The reaction mixture was extracted with ethyl acetate after the addition of water. The organic layer was washed with brine, dried and concentrated to obtain a crude body of a side-chain cyclization compound.
   The crude body of a side-chain cyclization compound:
   ¹H NMR (CDCl₃) δ : 0.06 (s, 12 H), 0.53 (s, 3 H), 0.88 (s, 18 H), 0.89-3.15 (m, 24 H), 4.19 (br., 1 H), 4.37 (br., 1 H), 4.86-4.96 (m, 2 H), 5.17 (s, 1 H), 5.48 (dd, J=7.1 and 15.3 Hz, 1 H), 5.63 (t, J=2.3 Hz, 1 H), 5.76-5.79 (m, 1 H), 6.02 (d, J=11.4 Hz, 1 H), 6.22-6.26 (m, 2 H).
   MS m/z 681.5 (M+1)⁺.

The obtained crude body of a side-chain cyclization compound was dissolved in a mixed solvent of acetonitrile (1 ml) and methylene chloride (1 ml), to the solution was added lithium tetrafluoroborate (25 mg, 270 µmol), and the solution was ice-cooled. To this solution was added an acetonitrile solution (162 µl, 1 N, 162 µmol) of sulfuric acid, and the mixture was stirred for 30 min under ice cooling. The reaction mixture was extracted with ethyl acetate after the addition of a saturated sodium hydrogencarbonate aqueous solution. The organic layer was washed with brine, dried and concentrated. The residue was purified by Sep-pak Plus Silica Cartridge manufactured by Waters Co. [the sample was dissolved in methylene chloride (2 ml), the sample solution was charged on the column, this was eluted with hexane : ethyl acetate=3:1 (10 ml) and successively with hexane : ethyl acetate: methanol=3:3:1 (10 ml), the hexane : ethyl acetate: methanol=3:3:1 fraction was collected, and this fraction was concentrated] to obtain a crude body of Compound No. 41 (38 mg). This crude compound was purified by HPLC (reversed phase, elution: A; 95% H₂O/CH₃CN, B; 95% MeOH/H₂O, B=81%) to obtain the objective Compound No. 41 (18.5 mg, 46% yield).
Compound No. 41:
¹H NMR (CDCl₃) δ : 0.55 (s, 3 H), 0.918 & 0.924 (d, J=6.6 Hz, 3 H), 1.28-2.07 (m, 15 H), 2.16 (br., 1 H), 2.32 (dd, J=6.8 and 13.7 Hz, 1 H), 2.57-2.73 (m, 2 H), 2.80-2.85 (m, 1 H), 3.08-3.18 (m, 1 H), 4.23 (br., 1 H), 4.44 (br., 1 H), 4.93 (quint, J=7.1 Hz, 1 H), 5.00 (s, 1 H), 5.33 (s, 1 H), 5.48 (dd, J=7.3 and 15.0 Hz, 1 H), 5.64 (t, J=2.5 Hz, 1 H), 5.73-5.84 (m, 1 H), 6.02 (d, J=11.1 Hz, 1 H), 6.24 (t, J=2.8 Hz, 1 H), 6.38 (d, J=11.4 Hz, 1 H).
MS m/z 453.3 (M+1)⁺.

### Example 11

### Production of 2(S)-methyl-20(R)-(2-(tetrahydro-3-vinylidene-2-furanon-5-yl)-1(E)-ethenyl)-9,10-secopregna-5(Z),7(E),10(19)-triene-1(S),3(R)-diol (Compound No. 32a and Compound No. 32b)

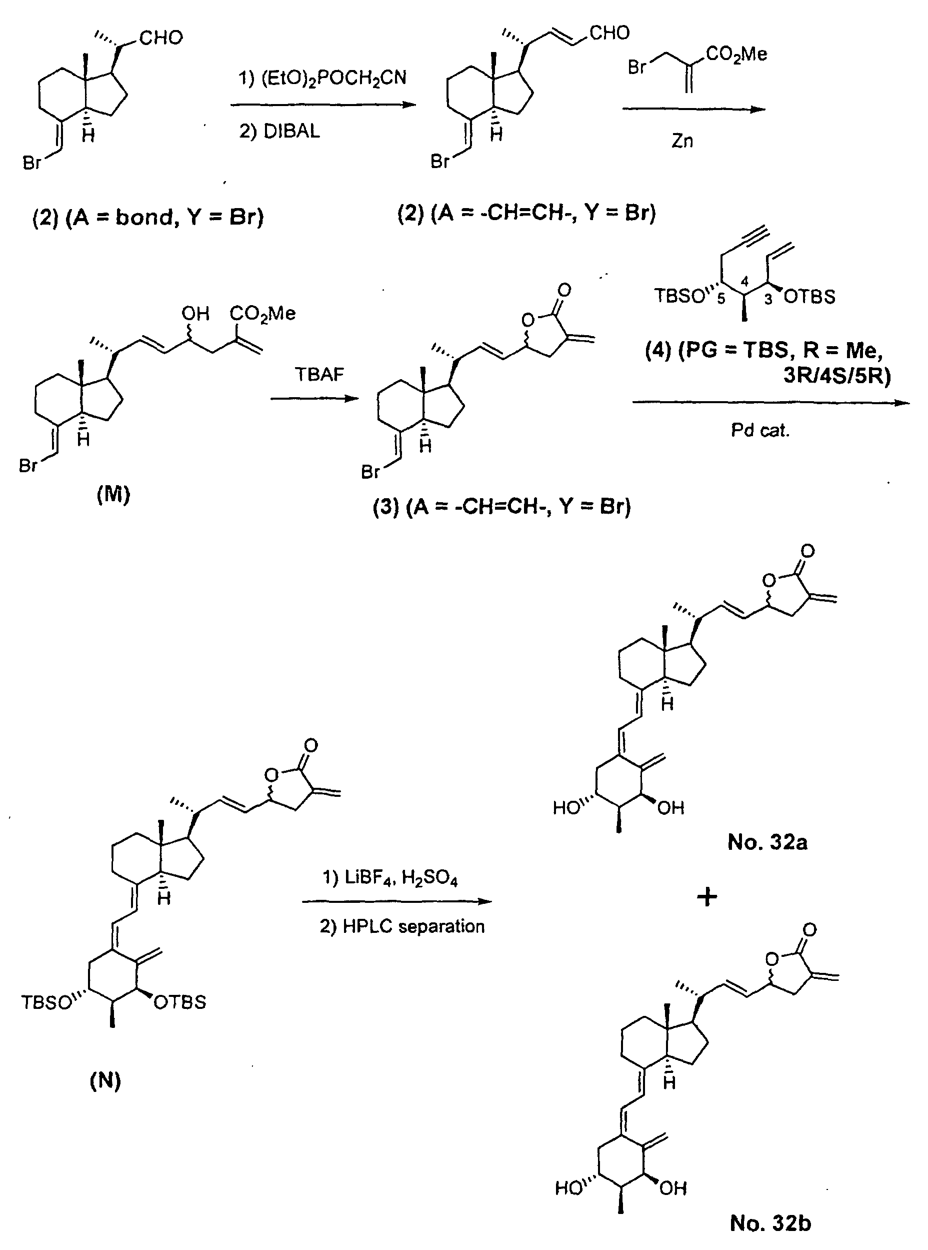

(1) Sodium hydride (108 mg, 60%, 2.55 mmol) was dissolved in anhydrous THF (10 ml), and the solution was ice-cooled. To the solution was added diethyl cyanomethylphosphonate (542 mg, 3.06 mmol), and the mixture was stirred for 2 hr under ice cooling. To the solution was added an anhydride THF solution (3 ml) of Compound (2) (A=single bond, Y=Br) (513 mg, 1.08 mmol), which can be obtained by a known method (for example, the method described in the specification of WO98/58909), the mixture was stirred for 2 hr while the temperature was gradually raised from ice-cooling temperature to room temperature. The reaction mixture was extracted with ethyl acetate after the addition of a saturated ammonium chloride aqueous solution. The organic layer was washed with brine, dried and concentrated to obtain a crude body (719 mg) of a Wittig adduct. The adduct was dissolved in anhydrous toluene (5 ml), and the solution was cooled to -60°C. To the solution was added a toluene solution (3.6 ml, 1.01 M, 3.6 mmol) of DIBAL, and the mixture was stirred for 3 hr at this temperature. The reaction mixture was extracted with ethyl acetate after the addition of methanol and 6 N hydrochloric acid, and the organic layer was washed with a saturated sodium hydrogencarbonate aqueous solution and brine, dried, and concentrated. The residue was purified by silica gel column chromatography (hexane : ethyl acetate=40:1 to 30:1) to obtain Compound (2) (A=-CH=CH-, Y=Br) (206 mg, 37% yield).
   Compound (2) (A=-CH=CH-, Y=Br):
   ¹H NMR (CDCl₃) δ: 0.62 (s, 3 H), 1.16 (d, J=6.8 Hz, 3 H), 1.23-1.86 (m, 9 H), 1.98-2.04 (m, 2 H), 2.39-2.48 (m, 1 H), 2.88-2.96 (m, 1 H), 5.67 (d, J=1.8 Hz, 1 H), 6.06 (dd, J=7.8 and 15.7 Hz, 1 H), 6.71 (dd, J=8.7 and 15.5 Hz, 1 H), 9.49 (d, J=7.8 Hz, 1 H).
   MS m/z 328.2 (M+1)⁺.
(2) The above-obtained Compound (2) (A=-CH=CH-, Y=Br) (206 mg, 0.66 mmol) was dissolved in anhydrous THF (5 ml), and the solution was ice-cooled. To the solution were added zinc powder (65 mg, 0.99 mmol) and methyl 2-bromomethylacrylate (178 mg, 0.99 mmol), and finally a saturated ammonium chloride solution (1 ml), and the mixture was stirred for 10 min under ice cooling and for 2 hr at room temperature. The reaction mixture was extracted with ethyl acetate after the addition of water. The organic layer was washed with brine, dried and concentrated. The residue was purified by silica gel column chromatography (hexane : ethyl acetate=20:1 to 5:1) to obtain Compound (M) (214 mg, 79% yield, colorless oil).
   Compound (M):
   ¹H NMR (CDCl₃) δ: 0.57 (s, 3 H), 1.02, 1.04 (d, J=6.8 and 6.6 Hz, 3 H), 1.23-1.75 (m, 9 H), 1.95-2.04 (m, 4 H), 2.48, 2.58 (dd, J=7.4 and 14.0 and 4.6 and 14.0 Hz, 2 H), 2.85-2.89 (m, 1 H), 3.77 (s, 3 H), 4.19-4.26 (m, 1 H), 5.35-5.57 (m, 2 H), 5.65 (d, J=4.3 Hz, 2 H), 6.25 (s, 1 H).
   MS m/z 392.9 (M-H₂O+1)⁺.
(3) The above-obtained Compound (M) (214 mg, 0.52 mmol) was dissolved in anhydrous THF (5 ml), and the solution was ice-cooled. To this solution was added a THF solution of tetrabutylammonium fluoride (1.56 ml, 1 N, 1.56 mmol), and the mixture was stirred for 2 hr under ice cooling. The reaction mixture was extracted with ethyl acetate after the addition of water. The organic layer was washed with brine, dried and concentrated. The residue was purified by silica gel column chromatography (hexane : ethyl acetate=10:1 to 5:1) to obtain Compound (3) (A=-CH=CH-, Y=Br) (147 mg, 74% yield, pale yellow oil).
   Compound (3) (A=-CH=CH-, Y=Br):
   ¹H NMR (CDCl₃) δ: 0.578, 0.582 (s, 3 H), 1.04-1.07 (m, 3 H), 1.24-1.71 (m, 9 H), 1.96-1.99 (m, 2 H), 2.05-2.14 (m, 1 H), 2.65-2.70 (m, 1 H), 2.86-2.90 (m, 1 H), 3.08-3.15 (m, 1 H), 4.89 (dd, J=6.6, 14.9 Hz, 1 H), 5.43 (dd, J=6.6 and 14.6 Hz, 1 H), 5.63-5.70 (m, 3 H), 6.24 (d, J=2.2 Hz, 1 H).
   MS m/z 379.0 (M+1)⁺.
(4) Pd₂(dba)₃ ·CHCl₃ (16 mg, 15 µmol) and triphenylphosphine (39 mg, 0.15 mmol) were dissolved in anhydrous toluene (1.0 ml) under a nitrogen atmosphere, and the mixture was stirred for 1 hr at room temperature. To the solution were added an anhydrous toluene solution (1.0 ml) of the above-obtained Compound (3) (A=-CH=CH-, Y=Br) (57 mg, 0.15 mmol) and Compound (4) (PG=TBS, R=Me, 3R/4S/5R), an ene-yne compound, (57 mg, 0.15 mmol), which had been manufactured by a method described in J. Med. Chem., 43, 4247-4265, (2000), and successively anhydrous triethylamine (2.0 ml); and the mixture was heated and stirred for 7.5 hr at 100°C. The reaction mixture was extracted with ethyl acetate after the addition of a saturated potassium sulfite aqueous solution. The organic layer was washed with brine, dried and concentrated, and the residue was purified by silica gel column chromatography (hexane : ethyl acetate=40:1 to 20:1) to obtain Compound (N) (43 mg, pale yellow oil).
   Compound (N):
   MS m/z 681.5 (M+1)⁺.
(5) The obtained Compound (N) (43 mg, 63 mmol) was dissolved in a mixed solvent of acetonitrile (1.5 ml) and methylene chloride (1.5 ml). To the solution was added lithium tetrafluoroborate (18 mg, 189 µmol), and the solution was ice-cooled. To the solution was added an acetonitrile solution (189 µl, 1 N, 189 µmol) of sulfuric acid, and the mixture was stirred for 30 min under ice cooling. Subsequently, the reaction temperature was raised to room temperature, further an acetonitrile solution (189 µl, 1 N, 189 µmol) of sulfuric acid was added every 45 min two times, and the mixture was stirred for 2.5 hr in total. The reaction mixture was extracted with ethyl acetate after the addition of a saturated sodium hydrogencarbonate aqueous solution. The organic layer was washed with brine, dried and concentrated. The residue was purified by Sep-pak Plus Silica Cartridge manufactured by Waters Co. [the sample was dissolved in methylene chloride (1.5 ml), the sample solution was charged on the column, this was eluted with hexane : ethyl acetate=3:1 (6 ml), hexane : ethyl acetate=1:1 (6 ml), and successively hexane : ethyl acetate: methanol=3:3:1 (12 ml), the hexane : ethyl acetate: methanol=3:3:1 fraction was collected, and the collected fraction was concentrated] to obtain a crude body (14 mg) of a mixture of Compound No. 32a and Compound No. 32b. The crude body was purified by HPLC (reversed phase, elution: A; 95% H₂O/CH₃CN, B; 95% MeOH/H₂O, B=78%) to obtain the objective Compound No. 32a (the lower polarity compound) (13 mg, 1.9% yield from Compound (3) (A=-CH=CH-, Y=Br)) in purity of 91% and Compound No. 32b (the higher polarity compound) (2.6 mg, 3.8% yield from Compound (3) (A=-CH=CH-, Y=Br)) in purity of 98%. Compound No. 32a (the lower polarity body) and Compound No. 32b (the higher polarity body) are diastereoisomers based on the asymmetric point on the lactone ring of the side-chain.
   Compound No. 32a (the lower polarity body):
   ¹H NMR (CDCl₃) δ: 0.55 (s, 3 H), 1.04 (d, J=6.6 Hz, 3 H), 1.08 (d, J=6.9 Hz, 3 H), 1.1-1.8 (m, 9 H), 1.8-2.0 (m, 3 H), 2.1-2.3 (m, 2 H), 2.62-2.71 (m, 2 H), 2.80-2.85 (m, 1 H), 3.06-3.15 (m, 1 H), 3.85 (br., 1 H), 4.31 (br., 1 H), 4.88 (q, J=7.3 Hz, 1 H), 5.00 (d, J=2.1 Hz, 1 H), 5.28 (s, 1 H), 5.41 (dd, J=7.3 and 15.3 Hz, 1 H), 5.63 (t, J=2.6 Hz, 1 H), 5.68 (dd, J=8.6 and 15.3 Hz, 1 H), 6.00 (d, J=11.2 Hz, 1 H), 6.23 (t, J=2.8 Hz, 1 H), 6.38 (d, J=10.9 Hz, 1 H).
   MS m/z 453.3 (M+1)⁺.
   Compound No. 32b (the higher polarity body):
   ¹H NMR (CDCl₃) δ: 0.55 (s, 3 H), 1.05 (d, J=7.3 Hz, 3 H), 1.08 (d, J=7.3 Hz, 3 H), 1.1-1.8 (m, 9 H), 1.8-2.1 (m, 3 H), 2.11-2.28 (m, 2 H), 2.65-2.69 (m, 2 H), 2.70-2.86 (m, 1 H), 3.07-3.16 (m, 1 H), 3.85 (br., 1 H), 4.31 (br., 1 H), 4.88 (q, J=7.1 Hz, 1 H), 5.00 (s, 1 H), 5.28 (s, 1 H), 5.41 (dd, J=8.7 and 15.8 Hz, 1 H), 5.63 (s, 1 H), 5.68 (dd, J=8.7 and 15.0 Hz, 1 H), 6.00 (d, J=11.7 Hz, 1 H), 6.23 (s, 1 H), 6.39 (d, J=11.4 Hz, 1 H).
   MS m/z 453.4 (M+1)⁺.

### Example 12

### Binding affinity to chick mucosal cell 1α, 25-dihydroxyvitamin D₃ receptor (VDR)

This receptor binding assay was performed as described by Ishizuka, et al. (Steroids, 37, 33-34 (1982)). That is, an ethanol solution (10 µl) of [26, 27-methyl-³H] 1α, 25-dihydroxyvitamin D₃ (15,000 dpm, 180 Ci/mmol) and an ethanol solution (40 µl) of a compound of the present invention were charged to a 12 × 75 mm polypropylene tube. Chick intestinal mucosal cell 1α, 25-dihydroxyvitamin D₃ receptor protein (0.2 mg) and gelatin (1 mg) were dissolved in 1 ml of phosphate buffer (pH 7.4), the solution was added to the tube, and the mixture was allowed to react for 1 hr at 25°C. One ml of a 40% polyethylene glycol 6000 solution was added to the tube, mixed vigorously, and then centrifuged (2,260 × g) for 60 min at 4°C. The bottom of the tube containing the pellet was cut off into a scintillation solution vial, 10 ml of dioxane-based scintillation fluid was added, and then radioactivity was measured by a liquid scintillation counter. Regarding compounds of the present invention, the concentration at which the binding of [26, 27-methyl-³H] 1α, 25-dihydroxyvitamin D₃ to the receptor was inhibited by 50% was determined from measured values. The concentration was expressed in terms of relative strength calculated by taking the 50%-inhibition concentration of 1α, 25-dihydroxyvitamin D₃ as 1. The results are shown in Table 2.

**Table 2**

| Binding affinities of compounds of the present invention to chick intestinal mucosal cell 1α, 25-dihydroxyvitamin D₃ receptor | |
|---|---|
| VDR affinity (1α, 25-dihydroxyvitamin D₃=1) | Compound No. |
| 1 to 1/5 | 31b, 32b, 41, 51b |
| 1/5 to 1/10 | 22d, 32a, 51a, 61, 71 |
| 1/10 to 1/30 | 22b, 31a |

Table 2 shows that compounds of the present invention have very strong binding affinities to VDR. Consequently, the compounds of the present invention can be expected to have high vitamin D3-like pharmacological activities, and it is suggested that they are effective as treating agents for various diseases, for example, osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia, Paget's disease of bone, etc.

### Examples 13

### Vitamin D₃ antagonist effect expressed by the parameter of differentiation induction effect on HL-60 cell caused by 1α,25-dihydroxyvitamin D₃

(1) HL-60 cell line which had been purchased from a cell bank (Japanese Cancer Research Resource Bank, Cell No: JCRB0085) was used. The cell line was stored as a frozen storage stock to prevent the change of cell characteristics attributable to successive cultivations. Prior to the initiation of experiments, the cells were defrosted and successive culturing was stared. For the experiments, cells whose successive culturing was from one month to about a half year were used. The successive culturing was carried out by centrifugally collecting cells which were in the state of suspension culture, and diluting the collected cell concentrate with a fresh culture medium at a ratio of about 1/100 (1-2 × 10⁴ cells/ml). As the culture medium, an RPMI-1640 medium containing 10% fetal bovine serum was used.
(2) The cells which were under successive culturing in the above process (1) were centrifugally collected, and they were dispersed in a culture medium at the cell concentration of 2×10⁴ cells/ml. The dispersion was seeded into a 24-well culture schale at 1 ml/well. Into this system, an ethanol solution which had been prepared by dissolving 1 α,25-dihydroxyvitamin D₃, or a compound of the present invention in ethanol so that it had a concentration of 1×10⁻⁵ M, or 1×10⁻⁶ M to 1×10⁻³ M, respectively, was added at 1 µl/well (the final concentration: 1×10⁻⁸ M in 1 α,25-dihydroxyvitamin D₃; and 1×10⁻⁹ M to 1×10⁻⁶ M in a compound of the present invention). As the control, ethanol was added at 1 µl/well. After culturing at 37°C for 4 days in the presence of 5% CO₂, the cells were centrifugally collected.
(3) As the parameter of differentiation inducing effect on HL-60 cells, the induction of nitroblue tetrazonium (henceforth, NBT) reduction activity was used. The NBT reduction activity was determined according to the following procedure. That is, centrifugally collected cells were suspended in a fresh culture medium, and subsequently NBT and 12-O-tetradecanoylphorbol-13-acetate were added in such a manner that their concentrations became 0.1% and 100 ng/ml, respectively. After the mixed suspension was incubated at 37°C for 25 min, a cytospin specimen was prepared. After air drying, it was stained with Kernechtrot, and the ratio of the positive cells of NBT reduction activity was determined under an optical microscope. Percentage ratios of the positive cell ratio in a simultaneous treatment with 1α,25-dihydroxyvitamin D₃ (1×10⁻⁸ M) and a compound of the present invention at various treating concentrations (1×10⁻⁹ to 1×10⁻⁶ M) to that in a single treatment with 1 α,25-dihydroxyvitamin D₃ (1×10⁻⁸ M) were plotted against treating concentrations of the compound of the present invention, and the treating concentration of the compound of the present invention at which the percentage ratio became 50% was determined as IC₅₀ value (nM). The results are shown in Table 3.

**Table 3**

| NBT reduction activity induction effect in HL-60 cell (suppression effect of compounds of the present invention on cell differentiation induction by 1α,25-dihydroxyvitamin D₃) | |
|---|---|
| IC₅₀ (nM) | Compound No. |
| <50 | 22b,22d,31b,32a |
| 50 to 150 | 11a, 31a, 32b, 51b |
| 150 to 300 | 51a, 61 |

Table 3 shows that compounds of the present invention suppress cell differentiation induction caused by 1α,25-dihydroxyvitamin D₃. That is, the compounds of the present invention act as antagonists against 1α,25-dihydroxyvitamin D₃. Consequently, it was suggested that the compounds of the present invention are effective as a treating agent for hypercalcemia and Paget's disease of bone, which are caused by the accentuation of activity of an active type vitamin D₃.

## Claims

1. Vitamin D₃ derivative expressed by the following general formula (1) 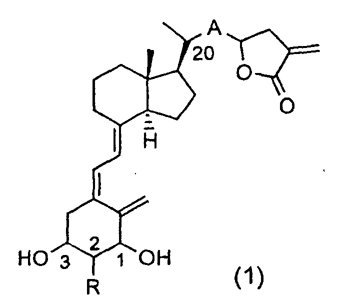 [wherein, R is a hydrogen atom or a methyl group, and A is a single bond, -CH₂-, -CH=CH-, -CH₂-CH=CH-, -CH=CH-CH=CH-, -C≡C- or -CH₂-C≡C-; and a compound in which R is an hydrogen atom, A is -CH₂-, the steric configuration at the 1-position is (S)-configuration, and the steric configuration at the 3-position is (R)-configuration is excluded] or a pharmaceutically permissible solvate thereof.

2. The vitamin D₃ derivative or its pharmaceutically permissible solvate thereof according to Claim 1, wherein the steric configuration of the 1-position is (S)-configuration, and that of the 3-position is (R)-configuration in the above formula (1).

3. The vitamin D₃ derivative or its pharmaceutically permissible solvate thereof according to Claim 1 or 2, wherein R is a methyl group in the above formula (1).

4. The vitamin D₃ derivative or its pharmaceutically permissible solvate thereof according to Claim 3, wherein the steric configuration of the 2-position is (S)-configuration in the above formula (1).

5. The vitamin D₃ derivative or its pharmaceutically permissible solvate thereof according to Claim 1 or 2, wherein R is a hydrogen atom in the above formula (1).

6. The vitamin D₃ derivative or its pharmaceutically permissible solvate thereof according to any one of the Claims 1 to 5, wherein A is -CH₂- or -CH=CH- in the above formula (1).

7. The vitamin D₃ derivative or its pharmaceutically permissible solvate thereof according to Claim 1, wherein the steric configuration of the 1-position is (S)-configuration, the steric configuration of the 3-position is (Reconfiguration, the steric configuration at the 20-position is (R)-conftguration, R is a hydrogen atom, and A is -CH=CH- in the above formula (1).

8. The vitamin D₃ derivative or its pharmaceutically permissible solvate thereof according to Claim 1, wherein the steric configuration of the 1-position is (S)-configuration, the steric configuration of the 2-position is (S)-configuration, the steric configuration at the 3-position is (R)-configuration, the steric configuration at the 20-position is (R)-configuration, R is a methyl group, and A is -CH=CH- in the above formula (1).

9. The vitamin D₃ derivative or its pharmaceutically permissible solvate thereof according to Claim 1, wherein the steric configuration of the 1-position is (S)-configuration, the steric configuration of the 2-position is (S)-configuration, the steric configuration at the 3-position is (R)-configuration, the steric configuration at the 20-position is (R)-configuration, R is a methyl group, and A is -CH₂- in the above formula (1).

10. The vitamin D₃ derivative or its pharmaceutically permissible solvate thereof according to Claim 1, wherein the steric configuration of the 1-position is (S)-configuration, the steric configuration of the 2-position is (S)-configuration, the steric configuration at the 3-position is (S)-configuration, the steric configuration at the 20-position is (R)-configuration, R is a methyl group, and A is -CH₂- in the above formula (1).

11. A treating agent for a disease selected from the group consisting of osteoporosis, malignant tumor, psoriasis, hyperparathyroidism, an inflammatory respiratory disease, rheumatoid arthritis, growth onset type diabetes mellitus, hypertension, alopecia, acne, dermatitis, hypercalcemia and Paget's disease of bone, containing a vitamin D₃ derivative or its pharmaceutically permissible solvate thereof according to any one of claims 1 to 10 in an effective amount for treatment.

12. The treating agent according to Claim 11, wherein the disease is hyperparathyroidism or Paget's disease of bone.

13. The treating agent according to Claim 11, wherein the disease is hyperparathyroidism.

14. The treating agent according to Claim 11, wherein the disease is Paget's disease of bone.

15. A pharmaceutical composition comprising a vitamin D₃ derivative or its pharmaceutically permissible solvate thereof according to any one of claims 1 to 10, and a pharmaceutically permissible support.
